# EUROPEAN PATENT APPLICATION

(11) **EP 3 689 914 A1**
(43) Date of publication of application: **05.08.2020**
(21) Application number: 18862750.9
(22) Date of filing: 01.10.2018
(51) Int. Cl.: C07K 19/00, C07K 14/62, A61K 47/54, A61K 47/68, A61K 38/00

(54) **PERSISTENT PROTEIN CONJUGATE WITH IMPROVED EFFICIENCY**

(30) Priority: 29.09.2017 KR 20170127418
(71) Applicant: Hanmi Pharm. Co., Ltd., Hwaseong-si, Gyeonggi-do 18536 (KR)
(72) Inventor: LEE, Jong-Soo, Hwaseong-si Gyeonggi-do 18469 (KR); KIM, Eun Jung, Hwaseong-si Gyeonggi-do 18469 (KR); KIM, Dae Jin, Hwaseong-si Gyeonggi-do 18469 (KR)
(74) Representative: Mewburn Ellis LLP
(86) International application number: PCT/KR2018/011616
(87) International publication number: WO 2019/066603

(57) **Abstract**

The present invention relates to a protein complex, in which a physiologically active polypeptide modified with a fatty acid molecule and an immunoglobulin Fc region are linked through a non-peptide polymer linker, a pharmaceutical composition comprising the same, and a method for preparing the protein complex. The protein complex extends the blood half-life of the physiologically active polypeptide through the linkage of the binding site of immunoglobulin Fc and the intramolecular bonding of the fatty acid molecule; has an excellent *in vivo* activity; and has no risk of inducing an immune response.

<110>    HANMI PHARM. CO., LTD.

<120>    A long acting protein complex having an enhanced efficiency

<130>    OPA18286-PCT

<150>    KR 10-2017-0127418
<151>    2017-09-29

<160>    18

<170>    KoPatentIn 3.0

<210>    1
<211>    258
<212>    DNA
<213>    Artificial Sequence

<220>
<223>    Analog 1


<400>    1
ttcgttaacc aacacttgtg tggctcacac ctggtggaag ctctctacct agtgtgcggg        60

gaacgaggct tcttctacac acccaagacc cgccgggagg cagaggacct gcaggtgggg       120

caggtggagc tgggcggggg ccctggtgca ggcagcctgc agcccttggc cctggagggg       180

tccctgcaga agcgtgcgat tgtggaacaa tgctgtacca gcatctgctc cctctaccag       240

ctggagaact actgcaac                                                     258


<210>    2
<211>    86
<212>    PRT
<213>    Artificial Sequence

<220>
<223>    Analog 1


<400>    2
Phe Val Asn Gln His Leu Cys Gly Ser His Leu Val Glu Ala Leu Tyr
  1               5                  10                  15

Leu Val Cys Gly Glu Arg Gly Phe Phe Tyr Thr Pro Lys Thr Arg Arg
             20                  25                  30

Glu Ala Glu Asp Leu Gln Val Gly Gln Val Glu Leu Gly Gly Gly Pro
         35                  40                  45

Gly Ala Gly Ser Leu Gln Pro Leu Ala Leu Glu Gly Ser Leu Gln Lys
     50                  55                  60

Arg Ala Ile Val Glu Gln Cys Cys Thr Ser Ile Cys Ser Leu Tyr Gln
 65                  70                  75                  80

Leu Glu Asn Tyr Cys Asn
                 85

<210>    3
<211>    258
<212>    DNA
<213>    Artificial Sequence

<220>
<223>    Analog 2


<400>    3
ttcgttaacc aacacttgtg tggctcacac ctggtggaag ctctctacct agtgtgcggg        60

gaacgaggct tcttctacac acccaagacc cgccgggagg cagaggacct gcaggtgggg       120

caggtggagc tgggcggggg ccctggtgca ggcagcctgc agcccttggc cctggagggg       180

tccctgcaga agcgtggcgc ggtggaacaa tgctgtacca gcatctgctc cctctaccag       240

ctggagaact actgcaac                                                     258


<210>    4
<211>    86
<212>    PRT
<213>    Artificial Sequence

<220>
<223>    Analog 2


<400>    4
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ala Val Glu Gln Cys Cys Thr Ser Ile Cys Ser Leu Tyr Gln
65                  70                  75                  80

Leu Glu Asn Tyr Cys Asn
                85


<210>    5
<211>    258
<212>    DNA
<213>    Artificial Sequence

<220>
<223>    Analog 3


<400>    5
ttcgttaacc aacacttgtg tggctcacac ctggtggaag ctctctacct agtgtgcggg        60

gaacgaggct tcttctacac acccaagacc cgccgggagg cagaggacct gcaggtgggg       120

caggtggagc tgggcggggg ccctggtgca ggcagcctgc agcccttggc cctggagggg 180

tccctgcaga agcgtggcat tgtggaacaa tgctgtacca gcatctgctc cctctaccag 240

ctggagaacg cgtgcaac 258

<210> 6
<211> 86
<212> PRT
<213> Artificial Sequence

<220>
<223> Analog 3

<400> 6
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Ala Cys Asn
                85

<210> 7
<211> 258
<212> DNA
<213> Artificial Sequence

<220>
<223> Analog 4

<400> 7
ttcgttaacc aacacttgtg tgcgtcacac ctggtggaag ctctctacct agtgtgcggg 60

gaacgaggct tcttctacac acccaagacc cgccgggagg cagaggacct gcaggtgggg 120

caggtggagc tgggcggggg ccctggtgca ggcagcctgc agcccttggc cctggagggg 180

tccctgcaga agcgtggcat tgtggaacaa tgctgtacca gcatctgctc cctctaccag 240

ctggagaact actgcaac 258

<210> 8
<211> 86
<212> PRT
<213> Artificial Sequence

<220>
<223>    Analog 4


<400>    8
Phe Val Asn Gln His Leu Cys Ala Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85


<210>    9
<211>    258
<212>    DNA
<213>    Artificial Sequence

<220>
<223>    Analog 5


<400>    9
ttcgttaacc aacacttgtg tggctcacac ctggtggaag ctctctacct agtgtgcggg      60

gaacgagcgt tcttctacac acccaagacc cgccgggagg cagaggacct gcaggtgggg      120

caggtggagc tgggcggggg ccctggtgca ggcagcctgc agcccttggc cctggagggg      180

tccctgcaga agcgtggcat tgtggaacaa tgctgtacca gcatctgctc cctctaccag      240

ctggagaact actgcaac                                                    258


<210>    10
<211>    86
<212>    PRT
<213>    Artificial Sequence

<220>
<223>    Analog 5


<400>    10
Phe Val Asn Gln His Leu Cys Gly Ser His Leu Val Glu Ala Leu Tyr
1               5                   10                  15

Leu Val Cys Gly Glu Arg Ala Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85


<210>    11
<211>    258
<212>    DNA
<213>    Artificial Sequence

<220>
<223>    Analog 6


<400>    11
ttcgttaacc aacacttgtg tggctcacac ctggtggaag ctctctacct agtgtgcggg        60

gaacgaggcg cgttctacac acccaagacc cgccgggagg cagaggacct gcaggtgggg       120

caggtggagc tgggcggggg ccctggtgca ggcagcctgc agcccttggc cctggagggg       180

tccctgcaga agcgtggcat tgtggaacaa tgctgtacca gcatctgctc cctctaccag       240

ctggagaact actgcaac                                                     258


<210>    12
<211>    86
<212>    PRT
<213>    Artificial Sequence

<220>
<223>    Analog 6


<400>    12
Phe Val Asn Gln His Leu Cys Gly Ser His Leu Val Glu Ala Leu Tyr
1               5                   10                  15

Leu Val Cys Gly Glu Arg Gly Ala Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85


<210>    13
<211>    258

<212>    DNA
<213>    Artificial Sequence

<220>
<223>    Analog 7


<400>    13
ttcgttaacc aacacttgtg tggctcacac ctggtggaag ctctctacct agtgtgcggg        60

gaacgaggct tcgcgtacac acccaagacc cgccgggagg cagaggacct gcaggtgggg       120

caggtggagc tgggcggggg ccctggtgca ggcagcctgc agcccttggc cctggagggg       180

tccctgcaga agcgtggcat tgtggaacaa tgctgtacca gcatctgctc cctctaccag       240

ctggagaact actgcaac                                                     258


<210>    14
<211>    86
<212>    PRT
<213>    Artificial Sequence

<220>
<223>    Analog 7


<400>    14
Phe Val Asn Gln His Leu Cys Gly Ser His Leu Val Glu Ala Leu Tyr
1               5                   10                  15

Leu Val Cys Gly Glu Arg Gly Phe Ala Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85


<210>    15
<211>    261
<212>    DNA
<213>    Artificial Sequence

<220>
<223>    Analog 8


<400>    15
ttcgttaacc aacacttgtg tggctcacac ctggtggaag ctctctacct agtgtgcggg        60

gaacgaggct tcttctacac acccaagacc cgccgggagg cagaggacct gcaggtgggg       120

caggtggagc tgggcggggg ccctggtgca ggcagcctgc agcccttggc cctggagggg       180

tccctgcaga agcgtggcat tgtggaacaa tgctgtacca gcatctgctc cctcgaacag 240

ctggagaact actgcaactg a 261

<210> 16
<211> 86
<212> PRT
<213> Artificial Sequence

<220>
<223> Analog 8

<400> 16
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Glu Gln
65                  70                  75                  80

Leu Glu Asn Tyr Cys Asn
                85

<210> 17
<211> 261
<212> DNA
<213> Artificial Sequence

<220>
<223> Analog 9

<400> 17
ttcgttaacc aacacttgtg tggctcacac ctggtggaag ctctctacct agtgtgcggg 60

gaacgaggct tcttctacac acccaagacc cgccgggagg cagaggacct gcaggtgggg 120

caggtggagc tgggcggggg ccctggtgca ggcagcctgc agcccttggc cctggagggg 180

tccctgcaga agcgtggcat tgtggaacaa tgctgtacca gcatctgctc cctcaaccag 240

ctggagaact actgcaactg a 261

<210> 18
<211> 86
<212> PRT
<213> Artificial Sequence

<220>
<223> Analog 9

<400>    18
Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Asn Gln
65                  70                  75                  80

Leu Glu Asn Tyr Cys Asn
                85

## Description

### [Technical Field]

The present invention relates to a protein complex, in which a physiologically active polypeptide modified with a fatty acid molecule and an immunoglobulin Fc region are linked through a non-peptide polymer linker; a pharmaceutical composition containing the same; and a method for preparing the protein complex. The protein complex extends the blood half-life of the physiologically active polypeptide through the linkage of the binding site of immunoglobulin Fc and the intramolecular bonding of the fatty acid molecule; has an excellent *in vivo* activity; and has no risk of inducing an immune response.

### [Background Art]

In general, polypeptides are easily denatured due to their low stability, degraded by proteases in the blood, and easily removed through the kidney or the liver. Therefore, in order to maintain the blood concentration and titer of a given protein drug containing a polypeptide as a pharmacologically active ingredient, frequent administration of the protein drug to patients is required. However, in the case of protein drugs administered to patients primarily in the form of an injectable formulation, frequent injections to maintain the blood concentration of active polypeptides may cause severe pain in patients. To solve these problems, efforts have been made to maximize the efficacy of protein drugs by increasing their blood stability and maintaining high blood drug concentrations for a long period of time. These long-acting protein drug formulations are required to increase their stability while simultaneously maintaining their activity at a sufficiently high level without causing immune responses in patients.

In the past, to stabilize proteins and inhibit their contact with proteases and loss through the kidney, a method for chemically adding polymers having a high solubility such as polyethylene glycol (hereinafter, abbreviated as "PEG") to the surface of protein drugs had been used. Although the method of linking PEG to the surface of protein drugs may have an advantage of increasing the stability of proteins, it also has problems in that the activities of physiologically active proteins are significantly lowered and that the reactivity with these proteins decreases as the molecular weight of PEG increases, resulting in a decrease in yield.

Meanwhile, immunoglobulin (Ig) largely includes a Fab region having an antigen binding site and an Fc region having a complement binding region. The methods for preparing a fusion protein by genetic recombination using the Fc region of a modified immunoglobulin are disclosed in U.S. Pat. Nos. 6,277,375, 6,410,008, 6,444,792, *etc.*

However, the Fc fusion proteins produced by such a genetic recombination method have disadavantages in that it is difficult to produce the Fc fusion proteins in the form of a monomer because protein fusions are allowed only at specific sites of an immunoglobulin Fc region (*i.e.,* at the amino- or carboxy terminus), that the Fc fusion proteins are expressed only in the form of a homodimer, and that only a fusion between glycosylated proteins or between aglycosylated proteins is possible and the fusion between a glycosylated protein and an aglycosylated protein is impossible. In addition, due to the amino acid sequence newly created by the fusion can cause not only an immune response, but also can increase the sensitivity of proteases to the linker region.

As such, the present inventors have previously confirmed that the method of preparing a complex using a linker has advantages in that the complex preparation can be controlled by selecting the binding sites and directionalities of the two proteins to be linked; monomers, dimers or multimers can be prepared; and both homo- and hetero forms can be prepared. In this regard, the present inventors have previously disclosed a method for producing a complex with a target protein in KR Patent Application Publication No. 10-2005-0047032, in which only a native immunoglobulin Fc region not containing a target protein is mass-produced from *E. coli* in high yield and used in long-acting formulations, and then, a complex with a target protein is produced using an E. coli-derived immunoglobulin Fc region, which is produced by such a recombination method, being mediated by a crosslinking agent. Further, the present inventors have confirmed in the above-mentioned patent application that a protein complex, which was prepared as a long-acting protein drug formulation capable of simultaneous achievement of both minimal activity reduction and stability enhancement, which had been conventionally considered difficult to achieve simultaneously, such that a glycosylated or non-glycosylated, native or genetically-recombined, immunoglobulin Fc region, a non-peptide polymer, and a physiologically active polypeptide were linked by a covalent bond with one another, significantly can increase the blood half-life of the physiologically active protein and maintain higher activities compared to known protein drugs.

However, in the case of protein complex drugs in which the shortcomings of physiologically active polypeptides are to be ameliorated, new improvements for a longer duration of activity and superior *in vivo* activity have been continuously required.

### [Disclosure]

### [Technical Problem]

An object of the present invention is to provide a protein complex, in which a physiologically active polypeptide modified with a fatty acid molecule and an immunoglobulin Fc region are linked through a non-peptide polymer linker.

Another object of the present invention is to provide a method of preparing the protein complex.

Still another object of the present invention is to provide a pharmaceutical composition with enhanced *in vivo* durability and stability, containing the protein complex as an active ingredient.

### [Technical Solution]

To achieve the above objects, an aspect of the present invention provides a protein complex, in which a physiologically active polypeptide modified with a fatty acid molecule and an immunoglobulin Fc region are linked through a non-peptide polymer linker.

In a specific embodiment, the physiologically active polypeptide modified with a fatty acid molecule is one in which at least one fatty acid molecule is linked to the physiologically active polypeptide, and the fatty acid molecule used in modification is characterized in that it is not linked to the immunoglobulin Fc region. In addition, the fatty acid molecule used in modification is characterized in that it is not a linker that links a physiologically active polypeptide and an immunoglobulin Fc region.

In another specific embodiment, the fatty acid molecule which has modified the physiologically active polypeptide is characterized in that it is a fatty acid or a fatty acid derivative.

In a specific embodiment according to any one of previous specific embodiments, the protein complex is characterized in that the non-peptide polymer linker, through reactive groups at both ends thereof, is linked to a physiologically active polypeptide modified with a fatty acid molecule and an immunoglobulin Fc region by a covalent bond, respectively.

In a specific embodiment according to any one of previous specific embodiments, the protein complex is characterized in that the immunoglobulin Fc region is aglycosylated.

In a specific embodiment according to any one of previous specific embodiments, the protein complex is characterized in that the immunoglobulin Fc region consists of one to four domains selected from the group consisting of CH1, CH2, CH3, and CH4 domains.

In a specific embodiment according to any one of previous specific embodiments, the protein complex is characterized in that the immunoglobulin Fc region further includes a hinge region.

In a specific embodiment according to any one of previous specific embodiments, the protein complex is characterized in that the immunoglobulin Fc region is an immunoglobulin Fc fragment selected from the group consisting of the Fc regions of IgG, IgA, IgD, IgE, IgM, a combination thereof, and a hybrid thereof.

In a specific embodiment according to any one of previous specific embodiments, the protein complex is characterized in that the immunoglobulin Fc region is selected from the group consisting of the Fc regions of IgG1, IgG2, IgG3, IgG4, a combination thereof, and a hybrid thereof.

In a specific embodiment according to any one of previous specific embodiments, the protein complex is characterized in that the immunoglobulin Fc fragment is a dimer or multimer consisting of single chain immunoglobulins consisting of domains having the same origin.

In a specific embodiment according to any one of previous specific embodiments, the protein complex is characterized in that the immunoglobulin Fc fragment is an IgG4 Fc fragment.

In a specific embodiment according to any one of previous specific embodiments, the protein complex is characterized in that the immunoglobulin Fc fragment is a human aglycosylated IgG4 Fc fragment.

In a specific embodiment according to any one of previous specific embodiments, the protein complex is characterized in that the non-peptide polymer linker is selected from the group consisting of polyethylene glycol, polypropylene glycol, an ethylene glycol-propylene glycol copolymer, polyoxyethylated polyol, polyvinyl alcohol, polysaccharide, dextran, polyvinyl ethyl ether, a biodegradable polymer, a lipid polymer, a fatty acid, a fatty acid derivative, chitin, hyaluronic acid, and a combination thereof.

In a specific embodiment according to any one of previous specific embodiments, the protein complex is characterized in that the non-peptide polymer linker is a fatty acid, a fatty acid derivative, or polyethylene glycol.

In a specific embodiment according to any one of previous specific embodiments, the protein complex is characterized in that the reactive group of the non-peptide polymer, which is a moiety constituting the complex, is selected from the group consisting of an aldehyde group, a maleimide group, and a succinimide derivative.

In a specific embodiment according to any one of previous specific embodiments, the protein complex is characterized in that the aldehyde group is a propionaldehyde group or butyraldehyde group.

In a specific embodiment according to any one of previous specific embodiments, the protein complex is characterized in that the succinimide derivative is succinimidyl carboxymethyl, succinimidyl valerate, succinimidyl methylbutanoate, succinimidyl methylpropionate, succinimidyl butanoate, succinimidyl propionate, N-hydroxysuccinimide, or succinimidyl carbonate.

In a specific embodiment according to any one of previous specific embodiments, the protein complex is characterized in that the non-peptide polymer linker has an aldehyde group, a maleimide reactive group, or a succinimide derivative as a reactive group at each end, respectively.

In a specific embodiment according to any one of previous specific embodiments, the protein complex is characterized in that the fatty acid molecule has an aldehyde group, a maleimide reactive group, or a succinimide derivative as a reactive group at each end, respectively.

In a specific embodiment according to any one of previous specific embodiments, the protein complex is characterized in that both ends of the non-peptide polymer linker are respectively linked to the N-terminus, a lysine residue, or an arginine residue of the immunoglobulin Fc region and the N-terminus, the C-terminus, a lysine residue, a histidine residue, or a cysteine residue of the physiologically active polypeptide; or a free reactive group of each terminus or residue thereof. The free reactive group is a reactive group which is not linked to any reactive group other than the reactive groups of the non-peptide polymer linker, and the free reactive group includes not only the reactive groups that are freed, but also the reactive groups capable of binding to the non-peptide polymer linker at each end or residue through modification, before binding to the non-peptide polymer linker, but the free reactive group is not limited thereto. In a specific embodiment according to any one of previous specific embodiments, the protein complex is characterized in that the physiologically active polypeptide is selected from the group consisting of hormone, cytokine, enzyme, antibody, growth factor, transcription factor, blood coagulation factor, vaccine, structural protein, ligand protein, and receptor.

In a specific embodiment according to any one of previous specific embodiments, the protein complex is characterized in that the physiologically active polypeptide is selected from the group consisting of human growth hormone, growth hormone-releasing hormone, growth hormone-releasing peptide, interferons, interferon receptors, colony-stimulating factors, glucagon-like peptides (GLP-1, *etc*.), exendins (Exendin-4, *etc*.), oxyntomodulin, G-protein-coupled receptor, interleukins, interleukin receptors, enzymes, interleukin-coupled proteins, cytokine-coupled proteins, macrophage activating factor, macrophage peptide, B cell factor, T cell factor, protein A, allergy-inhibiting factor, cell necrosis glycoprotein, immunotoxin, lymphotoxin, tumor necrosis factor, tumor-inhibiting factor, transforming growth factor, α-1 antitrypsin, albumin, α-lactalbumin, apolipoprotein-E, erythropoietin, high-glycosylated erythropoietin, angiopoeitins, hemoglobin, thrombin, thrombin receptor-activating peptide, thrombomodulin, blood coagulation factors VII, VIIa, VIII, IX, and XIII, plasminogen activator, fibrin-binding peptide, urokinase, streptokinase, hirudin, protein C, C-reactive protein, renin inhibitor, collagenase inhibitor, superoxide dismutase, leptin, platelet-derived growth factor, epithelial growth factor, epidermal growth factor, angiostatin, angiotensin, bone morphogenetic growth factor, bone morphogenetic-stimulating protein, calcitonin, insulin, atriopeptin, cartilage-inducing factor, elcatonin, connective tissue-activating factor, tissue factor pathway inhibitor, follicle-stimulating hormone, luteinizing hormone, luteinizing hormone-releasing hormone, nerve growth factors, parathyroid hormone, relaxin, secretin, somatomedin, insulin-like growth factor, adrenocortical hormone, glucagon, cholecystokinin, pancreatic polypeptide, gastrin-releasing peptide, corticotropin-releasing factor, thyroid-stimulating hormone, autotaxin, lactoferrin, myostatin, receptors, receptor antagonist, cell surface antigen, virus-derived vaccine antigen, monoclonal antibody, polyclonal antibody, antibody fragments, and a derivative thereof.

In a specific embodiment according to any one of previous specific embodiments, the protein complex is characterized in that the physiologically active polypeptide is selected from the group consisting of human growth hormone, interferon-alpha, granulocyte colony-stimulating factor, erythropoietin, blood coagulation factor, insulin, oxyntomodulin, glucagon-like peptides, exendins, and a derivative or analog thereof.

In a specific embodiment according to any one of previous specific embodiments, the protein complex is characterized in that the physiologically active polypeptide is insulin or an analog thereof, in which the fatty acid molecule which has modified the physiologically active polypeptide is a fatty acid or fatty acid derivative, and the non-peptide polymer linker is polyethylene glycol.

In a specific embodiment according to any one of previous specific embodiments, the protein complex is characterized in that the insulin analog is one in which any one, which is selected from the group consisting of the 8^{th} amino acid of insulin B-chain, the 23^{rd} amino acid of insulin B-chain, the 24^{th} amino acid of insulin B-chain, the 25^{th} amino acid of insulin B-chain, the 1^{st} amino acid of insulin A-chain, the 2^{nd} amino acid of insulin A-chain, and the 19^{th} amino acid of insulin A-chain, is substituted with alanine; or the 14^{th} amino acid of insulin A-chain is substituted with glutamic acid or asparagine.

To achieve the above objects, another aspect of the present invention provides a method of preparing a protein complex, in which the physiologically active polypeptide modified with a fatty acid molecule and an immunoglobulin Fc region are linked through a non-peptide polymer linker.

To achieve the above objects, still another aspect of the present invention provides a method of preparing a protein complex, which includes:
(a) linking a physiologically active polypeptide, which is in a form where at least one fatty acid molecule is linked thereto, and at least one immunoglobulin Fc region by a covalent bond through a non-peptide polymer linker having a reactive group at both ends to prepare a protein complex; and
(b) separating the protein complex prepared in Step (a), which essentially includes a physiologically active polypeptide, which is in a form where a fatty acid molecule is linked thereto, and an immunoglobulin Fc region that are linked by a covalent bond through the non-peptide polymer linker, wherein the non-peptide polymer linker is linked to the immunoglobulin Fc fragment.

In a specific embodiment, the above method of preparing a protein complex is characterized in that Step (a) includes:
(a1) linking one end of the non-peptide polymer linker to any one of the immunoglobulin Fc region and the physiologically active polypeptide, which is in a form where a fatty acid molecule is linked thereto, by a covalent bond to prepare a protein conjugate; and
(a2) separating the conjugate prepared in Step (a1) and linking the other end of the physiologically active polypeptide, which is in a form where a fatty acid molecule is linked thereto, of the separated conjugate to the other of the immunoglobulin Fc region and the physiologically active polypeptide, by a covalent bond.

In a specific embodiment according to any one of previous specific embodiments, the above method of preparing a protein complex is characterized in that in Step (a1), the reaction molar ratio between the physiologically active polypeptide, which is in a form where a fatty acid molecule is linked thereto, and the non-peptide polymer linker is in a range of 1:1 to 1:30, and the reaction molar ratio between the immunoglobulin Fc fragment and the non-peptide polymer is in a range of 1:1 to 1:20.

In a specific embodiment according to any one of previous specific embodiments, the above method of preparing a protein complex is characterized in that Step (a1) is performed at a pH between 4.0 and 9.0.

In a specific embodiment according to any one of previous specific embodiments, the above method of preparing a protein complex is characterized in that Step (a1) is performed at a temperature between 4.0°C and 25°C.

In a specific embodiment according to any one of previous specific embodiments, the above method of preparing a protein complex is characterized in that, in Step (a1), the reaction concentration of the immunoglobulin Fc region or the physiologically active polypeptide, which is in a form where a fatty acid molecule is linked thereto, is in a range of 0.1 mg/mL to 100 mg/mL.

In a specific embodiment according to any one of previous specific embodiments, the above method of preparing a protein complex is characterized in that, in Step (a2), the reaction molar ratio between the conjugate and the immunoglobulin Fc region or the physiologically active polypeptide is in a range of 1:0.1 to 1:20.

In a specific embodiment according to any one of previous specific embodiments, the above method of preparing a protein complex is characterized in that Step (a2) is performed at a pH between 4.0 and 9.0.

In a specific embodiment according to any one of previous specific embodiments, the above method of preparing a protein complex is characterized in that Step (a2) is performed at a temperature between 4.0°C and 25°C.

In a specific embodiment according to any one of previous specific embodiments, the above method of preparing a protein complex is characterized in that, in Step (a2), the reaction concentration of the immunoglobulin Fc region or the physiologically active polypeptide, which is in a form where a fatty acid molecule is linked thereto, is in a range of 0. 1 mg/mL to 100 mg/mL.

In a specific embodiment according to any one of previous specific embodiments, the above method of preparing a protein complex is characterized in that Step (a1) and Step (a2) are performed in the presence of a reducing agent.

In a specific embodiment according to any one of previous specific embodiments, the above method of preparing a protein complex is characterized in that the reducing agent is selected from the group consisting of sodium cyanoborohydride (NaCNBH₃), sodium borohydride, dimethylamine borate, and pyridine borate.

In a specific embodiment according to any one of previous specific embodiments, the above method of preparing a protein complex is characterized in that, in Step (a2), the conjugate is separated by performing a single purification method or a combination of multiple purification methods selected from the group consisting of anion exchange chromatography, cation exchange chromatography, hydrophobic chromatography, affinity chromatography, and size exclusion chromatography.

In a specific embodiment according to any one of previous specific embodiments, the above method of preparing a protein complex is characterized in that the functional group of the anion exchange chromatography resin is any one selected from the group consisting of quaternary ammonium (Q), quaternary aminoethyl (QAE), diethylaminoethyl (DEAE), polyethylene imine (PEI), dimethylaminomethyl (DMAE), and trimethylaminoethyl (TMAE).

In a specific embodiment according to any one of previous specific embodiments, the above method of preparing a protein complex is characterized in that the functional group of the cation exchange chromatography resin is any one selected from the group consisting of methylsulfonate (S), sulfopropyl (SP), carboxymethyl (CM), polyaspartic acid, sulfoethyl (SE), sulfopropyl (SP), phosphate (P), and sulfonate (S).

In a specific embodiment according to any one of previous specific embodiments, the above method of preparing a protein complex is characterized in that the functional group of the hydrophobic chromatography resin is any one selected from the group consisting of phenyl, octyl, (iso)propyl, butyl, and ethyl.

In a specific embodiment according to any one of previous specific embodiments, the above method of preparing a protein complex is characterized in that the functional group of the affinity chromatography resin is any one selected from the group consisting of protein A, heparin, blue, benzamidine, metal ions (cobalt, nickel, and copper), and an antibody to a part or the entirety of constituents of the protein complex, in which both ends of the physiologically active polypeptide, which is in a form where a fatty acid molecule is linked thereto, are respectively linked to the immunoglobulin Fc region and the physiologically active polypeptide.

In a specific embodiment according to any one of previous specific embodiments, the above method of preparing a protein complex is characterized in that the resin of the size exclusion chromatography is any one selected from the group consisting of Superdex, Sephacryl, Superpose, and Sephadex.

In a specific embodiment according to any one of previous specific embodiments, the above method of preparing a protein complex is characterized in that, in Step (b), the protein complex is separated by performing a single purification method or a combination of multiple purification methods selected from the group consisting of anion exchange chromatography, cation exchange chromatography, hydrophobic chromatography, affinity chromatography, and size exclusion chromatography.

In a specific embodiment according to any one of previous specific embodiments, the above method of preparing a protein complex is characterized in that the functional group of the anion exchange chromatography resin is any one selected from the group consisting of quaternary ammonium (Q), quaternary aminoethyl (QAE), diethylaminoethyl (DEAE), polyethylene imine (PEI), dimethylaminomethyl (DMAE), and trimethylaminoethyl (TMAE).

In a specific embodiment according to any one of previous specific embodiments, the above method of preparing a protein complex is characterized in that the functional group of the cation exchange chromatography resin is any one selected from the group consisting of methylsulfonate (S), sulfopropyl (SP), carboxymethyl (CM), polyaspartic acid, sulfoethyl (SE), sulfopropyl (SP), phosphate (P), and sulfonate (S).

In a specific embodiment according to any one of previous specific embodiments, the above method of preparing a protein complex is characterized in that the functional group of the hydrophobic chromatography resin is any one selected from the group consisting of phenyl, octyl, (iso)propyl, butyl, and ethyl.

In a specific embodiment according to any one of previous specific embodiments, the above method of preparing a protein complex is characterized in that the functional group of the affinity chromatography resin is any one selected from the group consisting of protein A, heparin, blue, benzamidine, metal ions (cobalt, nickel, and copper), and an antibody to a part or the entirety of constituents of the protein complex, in which both ends of the non-peptide polymer are respectively linked to the immunoglobulin Fc region and the physiologically active polypeptide.

In a specific embodiment according to any one of previous specific embodiments, the above method of preparing a protein complex is characterized in that the resin of the size exclusion chromatography is any one selected from the group consisting of Superdex, Sephacryl, Superpose, and Sephadex.

In a specific embodiment according to any one of previous specific embodiments, the above method of preparing a protein complex is characterized in that, in Step (b), the protein complex, in which a physiologically active polypeptide, which is in a form where a fatty acid molecule is linked thereto, is linked to an immunoglobulin Fc region through the N-terminus of the immunoglobulin Fc region, is separated.

To achieve the above objects, still another aspect of the present invention provides a method of preparing a protein complex, in which the physiologically active polypeptide modified with a fatty acid molecule and an immunoglobulin Fc region are linked through a non-peptide polymer linker, including:
(a') linking one end of the non-peptide polymer to any one of the immunoglobulin Fc region and the physiologically active polypeptide, which is in a form where a fatty acid molecule is linked thereto, by a covalent bond to prepare a conjugate,
   wherein the reaction molar ratio between the physiologically active polypeptide, which is in a form where a fatty acid molecule is linked thereto, and the non-peptide polymer is in a range of 1:1 to 1:30; the reaction molar ratio between the immunoglobulin Fc region and the non-peptide polymer linker is in a range of 1:1 to 1:20; a reducing agent is contained at a concentration of 1 mM to 100 mM; the reaction is performed at a pH between 4.0 and 9.0 and at a temperature between 4.0°C and 25°C; and the reaction concentration of the immunoglobulin Fc region or the physiologically active polypeptide is in a range of 0.1 mg/mL to 100 mg/mL;
(b') separating the conjugate prepared in Step (a') and linking the other end of the non-peptide polymer linker of the separated conjugate to the other of the immunoglobulin Fc region and the physiologically active polypeptide, by a covalent bond,
   wherein the reaction molar ratio between the conjugate and the immunoglobulin Fc region or the physiologically active polypeptide is in a range of 1:0.1 to 1:20; a reducing agent is contained at a concentration of 1 mM to 100 mM; the reaction is performed at a pH between 4.0 and 9.0 and at a temperature between 4.0°C and 25°C; and the reaction concentration of the immunoglobulin Fc region or the physiologically active polypeptide, which is in a form where a fatty acid molecule is linked thereto, is in a range of 0.1 mg/mL to 100 mg/mL; and
(c') separating the protein complex prepared in Step (b'), which essentially comprises a physiologically active polypeptide, which is in a form where a fatty acid molecule is linked thereto, a non-peptide polymer linker, and an immunoglobulin Fc region that are linked by a covalent bond, in which the non-peptide polymer linker is linked to the immunoglobulin Fc.

To achieve the above objects, still another aspect of the present invention provides a composition containing a protein complex, in which the physiologically active polypeptide modified with a fatty acid molecule and an immunoglobulin Fc region are linked through a non-peptide polymer linker.

In a specific embodiment, the composition is characterized in that it is a pharmaceutical composition for improving *in vivo* durability and stability of a physiologically active polypeptide.

### [Advantageous Effects]

The present invention provides a novel type of protein complex, in which a physiologically active polypeptide modified with a fatty acid molecule (*i.e*., a physiologically active polypeptide to which at least one fatty acid molecule is linked) and an immunoglobulin Fc region are linked through a non-peptide polymer linker, and a method of preparing the same. The protein complex of the present invention is characterized by having remarkably excellent *in vivo* durability and activity, compared to the existing protein complexes. Therefore, the protein complex prepared by the present invention can be effectively used for the development of long-acting formulations of a variety of physiologically active polypeptide drugs.

### [Brief Description of Drawings]

FIG. 1a is an image illustrating the results of the prepared (PBA-F)-fatty acid-native insulin-immunoglobulin Fc complex confirmed by SDS-PAGE.
FIG. 1b is an image illustrating the results of the prepared (PBA-F)-fatty acid-insulin analog-immunoglobulin Fc complex confirmed by SDS-PAGE.
FIG. 2a shows graphs illustrating the purity of the prepared (PBA-F)-fatty acid-native insulin-immunoglobulin Fc complexes confirmed by RP-HPLC, IE-HPLC, and SE-HPLC analyses, in which X-axis represents min and Y-axis represents mAU.
FIG. 2b shows graphs illustrating the purity of the prepared (PBA-F)-fatty acid-insulin analog-immunoglobulin Fc complexes confirmed by RP-HPLC, IE-HPLC, and SE-HPLC analyses, in which X-axis represents min and Y-axis represents mAU.
FIG. 3 is a graph comparing the hypoglycemic effect between the prepared long-acting insulin complexes and insulin analog complexes in diabetic model mice.
FIG. 4 is a schematic diagram of Ins-PBA-F.

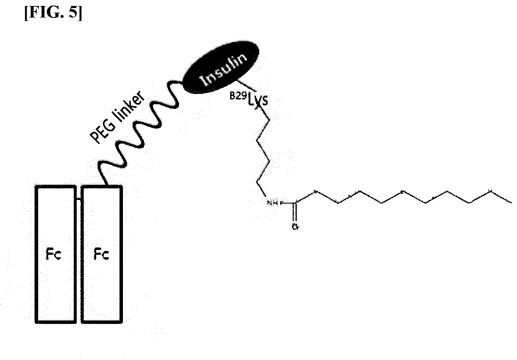

FIG. 5 is a schematic diagram of the protein complex of the present invention, in which a physiologically active polypeptide (insulin) modified with a fatty acid molecule and an immunoglobulin Fc region are linked to a non-peptide polymer linker (polyethylene glycol).

### [Best Mode for Carrying Out the Invention]

An aspect of the present invention provides a protein complex, in which a physiologically active polypeptide modified with a fatty acid molecule and an immunoglobulin Fc region are linked through a non-peptide polymer linker. Specifically, the physiologically active polypeptide modified with a fatty acid molecule is one in which at least one fatty acid molecule is linked to the physiologically active polypeptide, and the fatty acid molecule used in modification is characterized in that it is not linked to an immunoglobulin Fc region. The fatty acid molecule used in modification is not a linker because it does not act as a linker, and the fatty acid molecule used in modification is characterized in that it is not linked to an immunoglobulin Fc region.

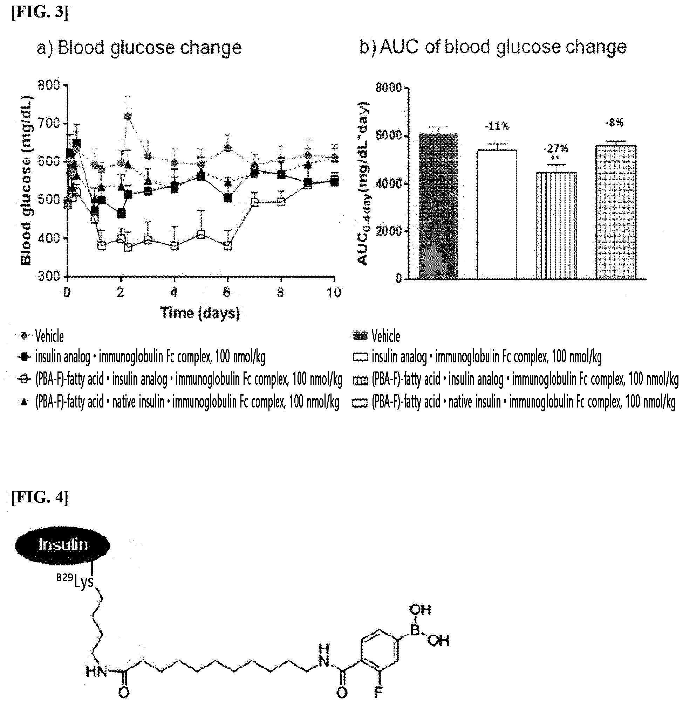

The present inventors have prepared a protein complex having a novel structure, in which a physiologically active polypeptide modified with a fatty acid molecule and an immunoglobulin Fc region are linked through a non-peptide polymer linker, the preparation and effects thereof had never been substantially confirmed, and have confirmed that the structure of the novel protein complex is superior, with regard to its durability and activity, to the existing protein complexes that had been used in protein drugs (Table 3 and FIG. 3). By such confirmation, the present inventors have developed a novel protein complex structure and provide the same herein as an aspect of the present invention.

The physiologically active polypeptide modified with a fatty acid molecule may be a physiologically active polypeptide which is in a form where at least one fatty acid molecule is linked thereto, but the form of the modified physiologically active polypeptide is not limited thereto.

As used herein, the term "protein complex" or "complex" refers to a structure in which at least one physiologically active polypeptide modified with a fatty acid molecule, at least one non-peptide polymer, and at least one immunoglobulin Fc region are respectively included as one moiety, and these constituents are linked to one another by a covalent bond. The non-peptide polymer may be one which has a reactive group at both ends to form a complex before forming the complex, but is not limited thereto. Further, a structure in which only two molecules selected from a physiologically active polypeptide, a fatty acid molecule, a non-peptide polymer, and an immunoglobulin Fc region are linked to each other by a covalent bond may be indicated as "conjugate", so as to distinguish the above structure from the structure of "complex".

The protein complex of the present invention may be one in which a non-peptide polymer linker, through its reactive group at both ends, is respectively linked to a physiologically active polypeptide, which is in a form where at least one fatty acid molecule is linked thereto, and an immunoglobulin Fc region, by a covalent bond.

As used herein, the term "physiologically active polypeptide", which may be a constitution constituting a moiety of the protein complex, is a general concept referring to polypeptides having a certain physiological activity *in vivo,* and it has the common feature to have a polypeptide structure and has various biological activities. The physiologically active polypeptide includes those having the role of correcting abnormal pathological conditions caused by deficiency or excessive secretion of materials, that are involved in regulation of functions *in vivo,* by adjusting genetic expression and physiological functions, and may also include general protein therapeutic agents. Additionally, the physiologically active polypeptide is a concept including not only native polypeptides but also all of the derivatives thereof.

As used herein, the term "physiologically active polypeptide", "physiologically active protein", "active protein", or "protein drug" refers to a polypeptide or protein exhibiting a certain antagonistic activity to a physiological event *in vivo,* and these terms may be used interchangeably.

As used herein, the term "fatty acid molecule", which may be a constitution constituting a moiety of the protein complex, refers to a fatty acid or fatty acid derivative, and the repeating units may be linked to each other by any covalent bond excluding a peptide bond, but is not limited thereto. Among the fatty acid molecules used in the modification, the fatty acid may include a monomeric fatty acid, a polymeric fatty acid formed by polymerization of monomeric fatty acids, and a derivative thereof, but the fatty acid molecules are not limited thereto.

As used herein, the term "fatty acid", which may be a constitution constituting a moiety of the complex, refers to a carboxylic acid of a hydrocarbon chain having one carboxy group (-COOH), and monovalent carboxylic acids having the formula of R-COOH are collectively referred to as fatty acid.

The fatty acid of the present invention may be a saturated fatty acid or unsaturated fatty acid depending on the bond between the carbon backbones that form a hydrocarbon chain. The unsaturated fatty acid refers to a fatty acid having at least one double bond in the bonds of the carbon backbone forming the hydrocarbon chain, and the saturated fatty acid refers to those in which all of the bonds of the carbon backbones consist of only single bonds.

In addition, the fatty acid of the present invention may be a fatty acid having a normal chain structure or may be a fatty acid having a side chain in an alkyl group. Fatty acids can be classified as short-chain/medium-chain/long-chain fatty acids according to the carbon number that forms a given hydrocarbon chain. In general, fatty acids can be classified as short-chain fatty acids (1 to 6 carbon atoms), medium-chain fatty acids (6 to 12 carbon atoms), and long-chain fatty acids (14 or more carbon atoms). In addition, in the case of unsaturated fatty acids, the characteristics may vary depending on the position of the double bond.

The fatty acid of the present invention may be a carboxylic acid having the formula of R-COOH, in which the R group may include a linear or branched saturated hydrocarbon group; a saturated fatty acid or unsaturated fatty acid; a short-chain fatty acid, a medium-chain fatty acid, or a long-chain fatty acid. Specifically, the fatty acid of the present invention may be a fatty acid having 1 to 40 carbon atoms, and more specifically 4 to 30 carbon atoms that forms a hydrocarbon, but the fatty acid of the present invention is not limited thereto. For example, the fatty acid of the present invention may be one selected from the group consisting of formic acid (HCOOH), acetic acid (CH3COOH), propionic acid (C₂H₅COOH), butyric acid (C₃H₇COOH), valeric acid (C₄H₉COOH), caproic acid (C₅H₁₁COOH), enanthic acid (C₆H₁₃COOH), caprylic acid (C₇H₁₅COOH), pelargonic acid (C₈H₁₇COOH), capric acid (C₉H₁₉COOH), undecylic acid (C₁₀H₂₁COOH), lauric acid (C₁₁H₂₃COOH), tridecylic acid (C₁₂H₂₅COOH), myristic acid (C₁₃H₂₇COOH), pentadecylic acid (C₁₄H₂₉COOH), palmitic acid (C₁₅H₃₁COOH), heptadecylic acid (C₁₆H₃₃COOH), stearic acid (C₁₇H₃₅COOH), nonadecanoic acid (C₁₈H₃₇COOH), arachidic acid (C₁₉H₃₉COOH), behenic acid (C₂₁H₄₃COOH), lignoceric acid (C₂₃H₄₇COOH), cerotic acid (C₂₅H₅₁COOH), heptacosanoic acid (C₂₆H₅₃COOH), montanic acid (C₂₈H₅₇COOH), melissic acid (C₂₉H₅₉COOH), lacceric acid (C₃₁H₆₃COOH), acrylic acid (CH₂=CHCOOH), crotonic acid (CH₃CH=CHCOOH), isocrotonic acid (CH₃CH=CHCOOH), undecylenic acid (CH₂=CH(CH₂)₈COOH), oleic acid (C₁₇H₃₃COOH), elaidic acid (C₁₇H₃₃COOH), cetoleic acid (C₂₁H₄₁COOH), erucic acid (C₂₁H₄₁COOH), brassidic acid (C₂₁H₄₁COOH), sorbic acid (C₅H₇COOH, F2), linoleic acid (C₁₇H₃₁COOH, F2), linolenic acid (C₁₇H₂₉COOH, F3), arachidonic acid (C₁₉H₃₁COOH, F4), propiolic acid (CH=CCOOH), hyaluronic acid (C₁₄H₂₁NO₁₁)ₙ), and stearolic acid (C₁₇H₃₁COOH, F1), but the fatty acid is not limited thereto.

In addition, the fatty acid of the present invention may be a derivative, analog, *etc.* of the fatty acids described above, and in particular, may be a variant in which the fatty acid-forming hydrocarbon includes a cyclic group in addition to a linear or branched group. The cyclic group which can be included in the hydrocarbon may be a saturated homocycle, heterocycle, aromatic condensed or non-condensed homocycle or heterocycle, and may have an ether bond, an unsaturated bond, and a substituent. For example, the fatty acid derivative may be a fatty acid to which a PBA compound is linked, but the fatty acid derivative is not limited thereto.

In addition, with regard to the fatty acid of the present invention, derivatives and analogs of the above fatty acids already known in the art, and derivatives and analogs which can easily be prepared within the state of the art also belong to the scope of the present invention. The molecular weight of the fatty acid of the present invention may be in a range of 0.1 kDa to 100 kDa, and specifically 0.1 kDa to 30 kDa. As the fatty acid of the present invention, not only a single kind of a polymer but a combination of different kinds of polymers may be used, but the fatty acid is not limited thereto.

As used herein, the term "non-peptide polymer", which may be a constitution constituting a moiety of the protein complex, refers to a biocompatible polymer including two or more repeating units that are linked to each other by any covalent bond excluding a peptide bond, but the non-peptide polymer is not limited thereto.

As used herein, the term "immunoglobulin Fc region", which may be a constitution constituting a moiety of the protein complex, refers to the remaining region of an immunoglobulin molecule, excluding the variable regions of the heavy and light chains, the heavy-chain constant region 1 (C_{H}1) and the light-chain constant region 1 (C_{L}1) of an immunoglobulin. The immunoglobulin Fc region may further include a hinge region at the heavy-chain constant region. In particular, the immunoglobulin Fc region of the present invention may be a fragment including a part or the entirety of the immunoglobulin Fc region, and in the present invention, an immunoglobulin Fc region may be used interchangeably with an immunoglobulin fragment.

A native Fc has a glycan at position Asn297 of the heavy-chain constant region 1, but E. coli-derived recombinant Fc is expressed in an aglycosylated form. The removal of a glycan from an Fc results in a decrease in binding affinity of Fc gamma receptors 1, 2, and 3 and complement (c1q) to the heavy-chain constant region 1, and thus the antibody-dependent cytotoxicity or complement-dependent cytotoxicity can be reduced or removed.

As used herein, the term "immunoglobulin constant region" may refer to an Fc fragment, which includes a heavy-chain constant region 2 (CH2) and a heavy-chain constant region 3 (CH3) (or including a heavy-chain constant region 4 (CH4)), excluding the variable regions of the heavy and light chains, a heavy-chain constant region 1 (CH1) and a light-chain constant region (CL) of an immunoglobulin, and may further include a hinge region at the heavy chain constant region. Additionally, the immunoglobulin constant region of the present invention, as long as it has a physiological function substantially equivalent to or better than the native type, may be an extended immunoglobulin constant region, which includes a part or the entirety of the immunoglobulin of the heavy-chain constant region 1 (CH1) and/or the light-chain constant region (CL), excluding the variable regions of the heavy and light chains of an immunoglobulin. Further, the immunoglobulin constant region of the present invention may be a region having a deletion in a relatively long part of the amino acid sequence corresponding to CH2 and/or CH3. That is, the immunoglobulin constant region of the present invention may include (1) a CH1 domain, a CH2 domain, a CH3 domain, and a CH4 domain, (2) a CH1 domain and a CH2 domain, (3) a CH1 domain and a CH3 domain, (4) a CH2 domain and a CH3 domain, (5) a combination of one or more domains of the constant region and an immunoglobulin hinge region (or a part of the hinge region), and (6) a dimer of each domain of the heavy-chain constant regions and a light-chain constant region. An immunoglobulin constant region including an immunoglobulin Fc fragment is safe to be used as a drug carrier because it is a biodegradable polypeptide that can be metabolized *in vivo.* In addition, an immunoglobulin Fc fragment is not only advantageous with regard to preparation, purification, and yield of a complex than an entire immunoglobulin molecule due to its relatively low molecular weight, but is also expected to increase the homogeneity of materials and reduce the risk of inducing blood antigenicity because an immunoglobulin Fc fragment is devoid of Fab, which shows high non-homogeneity due to the difference in amino acid sequence among different antibodies.

Meanwhile, the immunoglobulin constant region may originate from humans or animals, such as cows, goats, pigs, mice, rabbits, hamsters, rats, guinea pigs, *etc.,* and may preferably originate from humans. In addition, the immunoglobulin constant region may be selected from the group consisting of constant regions derived from IgG, IgA, IgD, IgE, IgM, or combinations or hybrids thereof, preferably derived from IgG or IgM, which are most abundant in the human blood, and most preferably derived from IgG, which is known to improve the half-life of ligand-binding proteins. In the present invention, the immunoglobulin Fc region may be a dimer or multimer consisting of single-chain immunoglobulins of domains of the same origin.

As used herein, the term "combination" means that polypeptides encoding a single chain immunoglobulin constant region (preferably an Fc region) of the same origin are linked to a single-chain polypeptide of a different origin to form a dimer or multimer. That is, a dimer or a multimer can be prepared from two or more fragments selected from the group consisting of Fc fragments of IgG Fc, IgA Fc, IgM Fc, IgD Fc, and IgE Fc.

As used herein, the term "hybrid" means that sequences encoding two or more immunoglobulin constant regions of different origins are present in a single-chain of an immunoglobulin constant region (preferably, an Fc region). In the present invention, various hybrid forms are possible. That is, it is possible that the hybrid domain may be composed of one to four domains selected from the group consisting of CH1, CH2, CH3, and CH4 of IgG Fc, IgM Fc, IgA Fc, IgE Fc, and IgD Fc, and may further include a hinge region.

IgG can also be divided into IgG1, IgG2, IgG3, and IgG4 subclasses, and in the present invention, IgG may include a combination or hybrid thereof, specifically IgG2 and IgG4 subclasses, and more specifically the Fc region of IgG4, which has almost no effector function (e.g., complement-dependent cytotoxicity (CDC)).

In addition, the immunoglobulin constant region may be in a form with natural glycosylation, increased or reduced glycosylation, or deglycosylation. Increased/decreased glycosylation or deglycosylation of the immunoglobulin constant region may be achieved by conventional methods (e.g., chemical methods, enzymatic methods, genetic engineering method using microorganisms). In particular, an immunoglobulin constant region with deglycosylation does not induce unnecessary immune responses *in vivo* because the binding affinity of the complement (C1q) is significantly reduced and the antibody-dependent cytotoxicity or complement-dependent cytotoxicity is reduced or removed. In this regard, the forms of the immunoglobulin constant region more suitable for its original purpose as a drug carrier may be those with deglycosylation or aglycosylation. Accordingly, more specifically, the immunoglobulin Fc region may be an aglycosylated Fc region derived from a human IgG4 (*i.e.,* a human IgG4-derived aglycosylated Fc region). The human-derived Fc region is more preferable to a non-human derived Fc region, which may act as an antigen in the human body and cause undesirable immune responses (*e.g.,* generation of a new antibody against the antigen, *etc*.).

Further, the immunoglobulin constant region of the present invention includes not only the native amino acid sequence but also sequence derivatives thereof. The amino acid sequence derivative refers to an amino acid sequence which is different in sequence from the wild-type amino acid sequence as a result of deletion, insertion, conserved or non-conserved substitution of one or more amino acid residues, or a combination thereof. For example, in the case of IgG Fc, the amino acid residues at positions 214 to 238, 297 to 299, 318 to 322, or 327 to 331, which are known to be important for linkage, may be used as the sites suitable for modification. Additionally, various other kinds of derivatives are possible, including one that has a deletion of a region capable of forming a disulfide bond, or a deletion of some amino acid residues at the N-terminus of native Fc, an addition of a methionine residue at the N-terminus of native Fc, *etc.* Further, to remove effector functions, deletion may occur in a complement-binding site (*e.g.,* a C1q-binding site or an antibody dependent cell mediated cytotoxicity (ADCC) site). Techniques of preparing such sequence derivatives of the immunoglobulin constant region are disclosed in International Patent Publication Nos. WO 97/34631, WO 96/32478, *etc.*

The amino acid exchanges in proteins and peptides which do not generally alter the activity of the molecules are known in the art (H. Neurath, R. L. Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, the immunoglobulin Fc region may be modified by phosphorylation, sulfation, acrylation, glycosylation, methylation, farnesylation, acetylation, amidation, *etc.*

The above-described immunoglobulin constant region derivatives may be those which show biological activity identical to that of the immunoglobulin constant region of the present invention but with improved structural stability against heat, pH, *etc.* Additionally, the immunoglobulin constant region may be obtained from native forms isolated *in vivo* from humans and animals (*e.g.,* cows, goats, pigs, mice, rabbits, hamsters, rats, guinea pigs, *etc.*) or may be recombinants or derivatives thereof, obtained from transgenic animal cells or microorganisms. In particular, the immunoglobulin constant region may be obtained from a native immunoglobulin by isolating a whole immunoglobulin from a living human or animal body and treating the isolated immunoglobulin with protease. When the whole immunoglobulin is treated with papain, it is cleaved into Fab and Fc regions, whereas when the whole immunoglobulin is treated with pepsin, it is cleaved into pF'c and F(ab)₂ fragments. These fragments may be subjected to size exclusion chromatography, *etc* to isolate Fc or pF'c.

Specifically, the human-derived immunoglobulin constant region may be a recombinant immunoglobulin constant region obtained from a microorganism, but is not limited thereto.

The protein complex of the present invention may include at least one unit structure of a [physiologically active polypeptide/non-peptide polymer linker/immunoglobulin Fc region], in which all constituents may be linked in a linear form by a covalent bond. The non-peptide polymer linker may have a reactive group at both ends thereof, and through the same, is linked to the physiologically active polypeptide and the immunoglobulin Fc region through the reactive group by a covalent bond, respectively. That is, at least one conjugate, where a physiologically active polypeptide (which is in a form where at least one fatty acid molecule is linked thereto) is linked to the non-peptide polymer linker, is linked to one immunoglobulin Fc region by a covalent bond, and thereby forms a monomer, dimer, or multimer of the physiologically active polypeptide, which is mediated by the immunoglobulin Fc region. As such, the improvement of *in vivo* activity and stability can be more effectively achieved.

The reactive group at both ends of the non-peptide polymer is preferably selected from the group consisting of a reactive aldehyde group, a propionaldehyde group, a butyraldehyde group, a maleimide group, and a succinimide derivative. In the above, the succinimide derivative may be hydroxy succinimidyl, succinimidyl carboxymethyl, succinimidyl valerate, succinimidyl methylbutanoate, succinimidyl methylpropionate, succinimidyl butanoate, succinimidyl propionate, N-hydroxysuccinimide, or succinimidyl carbonate. In particular, when the non-peptide polymer has a reactive aldehyde group at both ends, it is effective in minimizing nonspecific reactions and linking, at both ends of the non-peptide polymer, to the physiologically active polypeptide and the immunoglobulin Fc region, respectively. The final product generated via reductive alkylation by an aldehyde bond is much more stable than when it is linked by an amide bond.

The reactive groups at both ends of the non-peptide polymer or the farry acid molecule of the present invention may be the same or different from each other. The non-peptide polymer or fatty acid molecule may have an aldehyde reactive group at both ends; may have an aldehyde group and a maleimide reactive group at each end, respectively; or an aldehyde group and a succinimide reactive group at each end, respectively, but the non-peptide polymer or fatty acid molecule is not limited thereto.

In an embodiment, the non-peptide polymer may have a maleimide group at one end and an aldehyde group, a propionaldehyde group, or a butyraldehyde group at the other end. In another embodiment, the non-peptide polymer may have a succinimidyl group at one end and a propionaldehyde group or butyraldehyde group at the other end. When a poly(ethylene glycol) having a reactive hydroxy group at both ends is used as a non-peptide polymer, the hydroxy group may be activated to various reactive groups by known chemical reactions, or a commercially available poly(ethylene glycol) having a modified reactive group may be used so as to prepare the protein complex of the present invention.

When the linkage between a physiologically active polypeptide (which is in a form where at least one fatty acid molecule is linked thereto) and an immunoglobulin Fc region is mediated by a non-peptide polymer linker, both ends of the non-peptide polymer may respectively bind to the N-terminus of the immunoglobulin Fc region and a free reactive group of the N-terminus (amino terminus), C-terminus (carboxy terminus), a lysine residue, a histidine residue, an arginine redisue or a cysteine residue of the physiologically active polypeptide.

The non-peptide polymer of the present invention may be selected from the group consisting of polyethylene glycol, polypropylene glycol, a copolymer of ethylene glycol and propylene glycol, polyoxyethylated polyol, polyvinyl alcohol, polysaccharide, dextran, polyvinyl ethyl ether, biodegradable polymers (*e.g*., polylactic acid (PLA) and polylactic-glycolic acid (PLGA)), lipid polymer, fatty acid, chitins, hyaluronic acid, and a combination thereof, and specifically, polyethylene glycol or fatty acid, but the non-peptide polymer is not limited thereto. Further, derivatives thereof which are well known in the art and can easily be prepared within the state of the art are included within the scope of the non-peptide polymer of the present invention. The non-peptide polymer may have a molecular weight in a range of 1 kDa to 100 kDa, and specifically 1 kDa to 20 kDa.

Examples of the physiologically active polypeptide of the present invention may include various physiologically active polypeptides (*e.g*., hormone, cytokine, interleukin, interleukin-binding protein, enzyme, antibody, growth factor, transcription factor, blood coagulation factor, vaccine, structural protein, ligand protein or receptor, cell surface antigen, receptor antagonist, and derivative or analog thereof), variants thereof and analogs thereof.

Specifically, examples of the physiologically active polypeptide may include various kinds, such as human growth hormone, growth hormone-releasing hormone, growth hormone-releasing peptide, interferons and interferon receptors (*e.g*., interferon-alpha, -beta, and -gamma, soluble type I interferon receptor, *etc.),* colony-stimulating factor, interleukins (*e.g.,* interleukin-1, -2, -3, -4, -5, -6, -7, -8, -9, -10, -11, -12, -13, -14, -15, -16, -17, -18, -19, -20, -21, -22, -23, -24, -25, -26, -27, -28, -29, -30, *etc*.) and interleukin receptors (*e.g.,* IL-1 receptor, IL-4 receptor, *etc*.), enzymes (e.g., glucocerebrosidase, iduronate-2-sulfatase, alpha-galactosidase-A, agalsidase alpha, beta, alpha-L-iduronidase, butyrylcholinesterase, chitinase, glutamate decarboxylase, imiglucerase, lipase, uricase, platelet-activating factor acetylhydrolase, neutral endopeptidase, myeloperoxidase, *etc*.), interleukin- and cytokine-binding proteins (*e.g.,* IL-18 bp, TNF-binding protein, *etc*.), macrophage-activating factor, macrophage peptide, B-cell factor, T-cell factors, protein A, allergy-inhibiting factor, cell necrosis glycoprotein, immunotoxin, lymphotoxin, tumor necrosis factor, tumor-inhibiting factor, transforming growth factor, alpha-1 anti-trypsin, albumin, alpha-lactalbumin, apolipoprotein-E, erythropoietin, high-glycosylated erythropoietin, angiopoietins, hemoglobin, thrombin, thrombin receptor activating peptide, thrombomodulin, blood coagulation factors VII, VIIa, VIII, IX, and XIII, plasminogen activator, fibrin-binding peptide, urokinase, streptokinase, hirudin, protein C, C-reactive protein, renin inhibitor, collagenase inhibitor, superoxide dismutase, leptin, platelet-derived growth factor, epithelial growth factor, epidermal growth factor, angiostatin, angiotensin, bone morphogenetic growth factor, bone morphogenetic-stimulating protein, calcitonin, insulin, oxyntomodulin, glucagon, glucagon derivative, glucagon-like peptide, exendins (Exendin-4), atriopeptin, cartilage-inducing factor, elcatonin, connective tissue-activating factor, tissue factor pathway inhibitor, follicle-stimulating hormone, luteinizing hormone, luteinizing hormone-releasing hormone, nerve growth factors (*e.g*., nerve growth factor, cilliary neurotrophic factor, axogenesis factor-1, brain-natriuretic peptide, glial-derived neurotrophic factor, netrin, neutrophil inhibitor factor, neurotrophic factor, neurturin, *etc*.), parathyroid hormone, relaxin, secretin, somatomedin, insulin-like growth factor, adrenocortical hormone, glucagon, cholecystokinin, pancreatic polypeptide, gastrin-releasing peptide, corticotrophin-releasing factor, thyroid-stimulating hormone, autotaxin, lactoferrin, myostatin, receptors (*e.g*., TNFR (P75), TNFR (P55), IL-1 receptor, VEGF receptor, B-cell-activating factor receptor, *etc*.), receptor antagonist (*e.g.,* IL1-Ra, *etc*.), cell surface antigen (*e.g.,* CD 2, 3, 4, 5, 7, 11a, 11b, 18, 19, 20, 23, 25, 33, 38, 40, 45, 69, *etc*.), monoclonal antibody, polyclonal antibody, antibody fragments (*e.g.,* scFv, Fab, Fab', F(ab')₂, and Fd), and virus-derived vaccine antigen, *etc.,* but are not limited thereto.

In particular, specifically, the physiologically active polypeptide of the present invention may be human growth hormone, interferons (interferon-alpha, -beta, -gamma, *etc*.) granulocyte colony-stimulating factor, erythropoietin, blood coagulation factors VII, VIIa, and VIII, insulin, oxyntomodulin, glucagon, glucagon-like peptides, exendins, antibody fragments, derivatives thereof, *etc.,* which are administered to the human body at high frequency for the purpose of treatment of prevention of disease. Additionally, certain variants or derivatives, or analogs thereof are included in the scope of the physiologically active polypeptide of the present invention as long as they have the function, structure, activity, or stability, which are substantially identical to or greater than those of native forms of the physiologically active polypeptides.

As used herein, the term "insulin" includes all of the peptides or modified peptides which have an insulin receptor-stimulating activity, for example, native insulin; a rapid-acting insulin; a basal insulin; an insulin analog in which any amino acid of native insulin is altered by any one method selected from substitution, addition, deletion, and modification, or by a combination of these methods; or a fragment thereof. Additionally, the insulin used in the present invention may be a long-acting insulin to which a long-acting technology is applied so as to overcome its short half-life. Preferably, the insulin may be a long-acting insulin or long-acting insulin analog which can be administered once a week. For example, the insulin may include the insulin or insulin analogs and long-acting types of insulin described in KR Patent No. 10-1058290, KR Patent Application Publication No. 10-2014-0106452, and International Patent Publication No. WO 2014/133324, but the scope of the insulin is not limited thereto.

As used herein, the term "insulin analog" refers to insulin in which at least amino acid in the native insulin sequence is modified.

The insulin analog may be an insulin analog with altered amino acid(s) in the A-chain or B-chain of insulin, in which reduced insulin activity and/or reduced insulin receptor binding affinity compared to the native insulin.

The insulin analog may be one in which any one, which is selected from the group consisting of the 8^{th} amino acid of insulin B-chain, the 23^{rd} amino acid of insulin B-chain, the 24^{th} amino acid of insulin B-chain, the 25^{th} amino acid of insulin B-chain, the 1^{st} amino acid of insulin A-chain, the 2^{nd} amino acid of insulin A-chain, and the 19^{th} amino acid of insulin A-chain, is substituted with alanine; or the 14^{th} amino acid of insulin A-chain is substituted with glutamic acid or asparagine, but the insulin analog is not limited thereto.

For example, the protein complex of the present invention may be one, in which the physiologically active polypeptide is insulin or an analog thereof, and the fatty acid molecule which has modified the physiologically active polypeptide is a fatty acid or fatty acid derivative, and the non-peptide polymer linker is polyethylene glycol, but the protein complex of the present invention is not limited thereto.

In the present invention, the antibody fragment may be Fab, Fab', F(ab')₂, Fd, or scFv having an ability to bind to a specific antigen, and preferably, Fab'. The Fab fragment includes a variable domain (VL) and a constant domain (CL) of the light chain and a variable domain (VH) and the first constant domain (CH1) of the heavy chain. The Fab' fragment differs from the Fab fragment in that several amino acid residues including at least one cysteine residue from a hinge region at the carboxyl terminus of the CH1 domain are added. The Fd fragment is a fragment consisting of only VH and CH1 domains, and the F(ab')₂ fragment is generated by a binding of two molecules of Fab' fragment by a disulfide bond or chemical reaction. The scFv fragment is a single polypeptide chain, in which only VL and VH domains are linked to each other by a peptide linker.

Further, the protein complex of the present invention may be used in the development of long-acting protein formulations of animal growth hormone (*e.g*., bovine growth hormone or porcine growth hormone) and long-acting protein formulations (*e.g*., interferon) for treatment or prevention of animal disease.

Still another aspect of the present invention provides a method of preparing a protein complex of the present invention. In particular, the present invention provides a method of preparing the protein complex, which includes: (a) linking a physiologically active polypeptide, which is in a form where at least one fatty acid molecule is linked thereto, and at least one immunoglobulin Fc region by a covalent bond through a non-peptide polymer linker having a reactive group at both ends to prepare a protein complex; and (b) separating the protein complex prepared in Step (a), which essentially includes a physiologically active polypeptide, which is in a form where a fatty acid molecule is linked thereto, and an immunoglobulin Fc region that are linked by a covalent bond through the non-peptide polymer linker, wherein the non-peptide polymer linker is linked to the immunoglobulin Fc fragment.

The immunoglobulin Fc region of the present invention may be in the form of a dimer or may be in the form of a homodimer or heterodimer. Therefore, the immunoglobulin Fc region constituting the protein complex of the present invention can be linked to at least one non-peptide polymer linker through an immunoglobulin Fc region. In particular, the immunoglobulin Fc region of the present invention may be in the form of a homodimer, and through the same, can be linked to a homodimer of the immunoglobulin Fc region through one or two non-peptide polymer linkers. In this case, each non-peptide polymer can bind to the physiologically active polypeptide, thereby forming a protein complex.

Accordingly, the protein complex of the present invention may be prepared such that one or two or more of a non-peptide polymer linker, one or two or more of a physiologically active polypeptide modified with a fatty acid molecule, and one or two or more of immunoglobulin Fc region are linked by a covalent bond.

In Step (a) above, the covalent bonds among the three constituents may occur sequentially or simultaneously. For example, when both the physiologically active polypeptide and the immunoglobulin Fc region are linked at both ends of the non-peptide polymer, respectively, it is advantageous to proceed with the reaction sequentially such that any one of the physiologically active polypeptide and the immunoglobulin Fc region is first linked to one end of the non-peptide polymer and then the other constituent is linked to the other end of the non-peptide polymer, so as to minimize the generation of by-products thereby preparing the desired protein complex in high purity.

Accordingly, Step (a) above may include: (i) linking a particular position of the immunoglobulin Fc region or the physiologically active polypeptide, which is in a form where a fatty acid molecule is linked thereto, to one end of the non-peptide polymer linker by a covalent bond; (ii) uniformly separating the conjugate, in which a particular position of the immunoglobulin Fc region or the physiologically active polypeptide is linked to the non-peptide polymer, from the reaction mixture; and (iii) producing a protein complex, in which the physiologically active polypeptide or a particular position of the immunoglobulin Fc region is linked to the other end of the non-peptide polymer of the separated conjugate.

Meanwhile, in the present invention, Step (a) above may include: (a1) linking one end of the non-peptide polymer to any one of the immunoglobulin Fc region and the physiologically active polypeptide, which is in a form where a fatty acid molecule is linked thereto, by a covalent bond to prepare a conjugate; and (a2) separating the conjugate prepared in Step (a1) and linking the other end of the non-peptide polymer of the separated conjugate to the other of the physiologically active polypeptide, which is in a form where a fatty acid molecule is linked thereto, and the immunoglobulin Fc region, by a covalent bond.

Specifically, Step (a) above may include: (a1') linking one end of the non-peptide polymer to the immunoglobulin Fc region by a covalent bond to prepare a conjugate; and (a2') separating the conjugate prepared in Step (a1') and linking the other end of the non-peptide polymer of the separated conjugate to the physiologically active polypeptide by a covalent bond.

Alternatively, Step (a) above may include: (a1") linking one end of the non-peptide polymer to the physiologically active polypeptide by a covalent bond to prepare a conjugate; and (a2") separating the conjugate prepared in Step (a1") and linking the other end of the non-peptide polymer of the separated conjugate to the immunoglobulin Fc region.

In the present invention, in Step (a1), Step (a1'), or Step (a1") described above, the reaction molar ratio between the physiologically active polypeptide, which is in a form where a fatty acid molecule is linked thereto, and the non-peptide polymer linker may be in a range of 1:1 to 1:30, and the reaction molar ratio between the immunoglobulin Fc region and the non-peptide polymer may be in a range of 1:1 to 1:20. Specifically, in Step (a1') above, the reaction molar ratio between the immunoglobulin Fc region and the non-peptide polymer may be in a range of 1:1 to 1:20, and specifically, in a range of 1:1 to 1:15, 1:1 to 1:10, or 1:1 to 1:4; and in Step (a1") above, the reaction molar ratio between the physiologically active polypeptide and the non-peptide polymer may be in a range of 1:1 to 1:30, and specifically, in a range of 1:1 to 1:15 or 1:1 to 1:10. The production yield and cost can be optimized according to the reaction molar ratio.

In addition, in the present invention, the above-described Step (a1), Step (a1'), or Step (a1") may be performed at a pH between 4.0 and 9.0; and the above-described Step (a1), Step (a1'), or Step (a1") may be performed at a temperature between 4.0°C and 25°C. In Step (a1), Step (a1'), or Step (a1") described above, the reaction concentration of the immunoglobulin Fc region or the physiologically active polypeptide may be in a range of 0.1 mg/mL to 100 mg/mL.

In the present invention, in Step (a2), Step (a2'), or Step (a2") described above, the reaction molar ratio between the conjuate and the immunoglobulin Fc region or the physiologically active polypeptide may be in a range of 1:0.1 to 1:20, and specifically, in a range of 1:0.2 to 1:10. Specifically, in Step (a2') above, the reaction molar ratio between the conjuate and the physiologically active polypeptide may be in a range of 1:0.1 to 1:20, and in Step (a2") above, the reaction molar ratio between the conjuate and the immunoglobulin Fc region may be in a range of 1: 0.1 to 1:20. The production yield and cost can be optimized according to the reaction molar ratio.

In addition, in the present invention, the above-described Step (a2), Step (a2'), or Step (a2") may be performed at a pH between 4.0 and 9.0; and the above-described Step (a2), Step (a2'), or Step (a2") may be performed at a temperature between 4.0°C and 25°C. In Step (a2), Step (a2'), or Step (a2") described above, the reaction concentration of the immunoglobulin Fc region or the physiologically active polypeptide may be in a range between 0.1 mg/mL and 100 mg/mL.

Meanwhile, the preparation method of the present invention may be a method of preparing a site-specific protein conjugate, which includes: (a') linking one end of the non-peptide polymer to any one of the immunoglobulin Fc region and the physiologically active polypeptide by a covalent bond to prepare a conjugate, wherein the reaction molar ratio between the physiologically active polypeptide and the non-peptide polymer is in a range of 1:1 to 1:30; the reaction molar ratio between the immunoglobulin Fc region and the non-peptide polymer is in a range of 1:1 to 1:20; a reducing agent is contained at a concentration of 1 mM to 100 mM; the reaction is performed at a pH between 4.0 and 9.0 and at a temperature between 4.0°C and 25°C; and the reaction concentration of the immunoglobulin Fc region or the physiologically active polypeptide is in a range between 0.1 mg/mL and 100 mg/mL; (b') separating the conjugate prepared in Step (a') and linking the other end of the non-peptide polymer of the separated conjugate to the other of the immunoglobulin Fc region or the physiologically active polypeptide, by a covalent bond, wherein the reaction molar ratio between the conjugate and the immunoglobulin Fc region or the physiologically active polypeptide is in a range of 1:0.1 to 1:20; a reducing agent is contained at a concentration of 1 mM to 100 mM; the reaction is performed at a pH between 4.0 and 9.0 and at a temperature between 4.0°C and 25°C; and the reaction concentration of the immunoglobulin Fc region or the physiologically active polypeptide is in a range of 0.1 mg/mL to 100 mg/mL; and (c') separating the protein complex prepared in Step (b'), which essentially comprises a physiologically active polypeptide, a non-peptide polymer, and an immunoglobulin Fc region that are linked by a covalent bond, in which the non-peptide polymer is linked to the N-terminus of the immunoglobulin Fc region, but the preparation method of the present invention is not limited thereto.

In the present invention, the reactions in the above-described Step (a1), Step (a1'), Step (a1"), Step (a2), Step (a2'), and Step (a2") may be performed in the presence of a reducing agent, considering the types of the reactive groups at both ends of the non-peptide polymer that participates in the reactions, as needed. The reducing agent of the present invention may be sodium cyanoborohydride (NaCNBH₃), sodium borohydride, dimethylamine borate, or pyridine borate. In particular, the concentration of the reducing agent (*e.g*., sodium cyanoborohydride), temperature and pH of the reaction solution, and total concentrations of the physiologically active polypeptide and the immunoglobulin Fc region participating in the reactions are important from the aspects of production yield and purity. To maximize the production of a high-purity uniform complex, a variety of combinations of the conditions are needed. Depending on the characteristics of the physiologically active polypeptide to be prepared, various conditions are possible, and the reducing agent (*e.g*., sodium cyanoborohydride) may be contained at a concentration between 1 mM and 100 mM, the reaction solution may be at a temperature between 0°C and 25°C, and at a pH condition between pH 4.0 and pH 9.0, and the concentration of the reaction protein (concentration of the immunoglobulin Fc region or physiologically active polypeptide included upon the reaction) may be in a range between 5 mg/mL and 100 mg/mL, but the conditions are not limited thereto.

Meanwhile, the separation of the conjugate in the above-described Step (a2), Step (a2'), and Step (a2") may be performed by a method appropriately selected from the conventional methods used in protein separation as needed, considering the characteristics (*e.g*., purity, hydrophobicity, molecular weight, and electric charge) required for the separated conjugate. For example, the separation may be performed by applying various known methods (*e.g*., size exclusion chromatography, hydrophobic chromatography, and ion exchange chromatography), and if necessary, a plurality of different methods may be used in combination to purify the conjugate with a higher purity.

According to the characteristics of the physiologically active polypeptide to be prepared, various conditions may be possible. Generally, to separate the conjugate in which an immunoglobulin Fc region or physiologically active polypeptide is linked to a non-peptide polymer, size exclusion chromatography may be the easiest method to apply. However, for future scale-up and separation of isomers generated by the linking of the non-peptide polymer to a position other than the desired position or a small amount of denatured products, which may be generated during preparation, it is also possible to use hydrophobic chromatography or ion exchange chromatography.

In the present invention, for final purification of a high-purity complex, Step (b) may be performed by a method appropriately selected from the conventional methods used in protein separation as needed, considering the characteristics (*e.g*., hydrophobicity, molecular weight, and electric charge). For example, the separation may be performed by applying various known methods including size exclusion chromatography, affinity chromatography, hydrophobic chromatography, and ion exchange chromatography, and if necessary, a plurality of different methods may be used in combination to purify the complex with a higher purity. Depending on the characteristics of the desired complex consisting of the physiologically active polypeptide, the non-peptide polymer, and the Fc constant region, various separation conditions may be possible. Generally, to separate the complex in which the physiologically active polypeptide and the immunoglobulin Fc region are respectively linked to both ends of the non-peptide polymer, size exclusion chromatography may be the easiest method to apply. However, for future scale-up and effective separation of isomers or side-reaction products generated by the linking of the physiologically active polypeptide or immunoglobulin Fc region and a non-peptide polymer at a position other than the desired position, or a small amount of denatured products, which may be generated during preparation, unreacted physiologically active polypeptides, non-peptide polymers, and immunoglobulin Fc regions, it is also possible to use hydrophobic chromatography, ion exchange chromatography, or affinity chromatography in combination. Specifically, hydrophobic chromatography and ion exchange chromatography may preferably be used in combination, and a combination into hydrophobic chromatography or a combination into ion exchange chromatography is also possible. Depending on the complex to be prepared, ion exchange chromatography or hydrophobic chromatography may be used alone, but the method is not limited thereto.

In the present invention, the ion exchange chromatography is a process that separates proteins based on the fact that proteins have an electric charge at a certain pH, and it allows proteins to pass through a charged ion resin-immobilized chromatography column and separate the proteins by a difference in their migration rate, and the ion exchange chromatography may be anion exchange chromatography or cation exchange chromatography.

The anion exchange chromatography is a process that uses a cation resin, and the functional group of the resin constituting the corresponding anion exchange chromatography may be any one selected from the group consisting of quaternary ammonium (Q), quaternary aminoethyl (QAE), diethylaminoethyl (DEAE), polyethylene imine (PEI), dimethylaminomethyl (DMAE), and trimethylaminoethyl (TMAE), but is not limited thereto.

In addition, the cation exchange chromatography is a process that uses an anion resin, and the functional group of the resin constituting the corresponding cation exchange chromatography may be any one selected from the group consisting of methylsulfonate (S), sulfopropyl (SP), carboxymethyl (CM), polyaspartic acid, sulfoethyl (SE), sulfopropyl (SP), phosphate (P), and sulfonate(S), but is not limited thereto.

In the present invention, the functional group of the resin constituting the hydrophobic chromatography may be any one selected from the group consisting of phenyl, octyl, (iso)propyl, butyl, and ethyl, but is not limited thereto.

In the present invention, the functional group of the resin constituting the size exclusion chromatography may be any one selected from the group consisting of Superdex, Sephacryl, Superpose, and Sephadex, but is not limited thereto.

Further, the affinity chromatography in the present invention is a process that separates particular proteins by a difference in their migration rate, according to the presence/absence of an interaction between the proteins and ligands capable of interacting with the proteins in a resin onto which the ligands are attached. The functional group of the resin constituting the affinity chromatography may be any one selected from the group consisting of protein A, heparin, blue, benzamidine, metal ions (cobalt, nickel, and copper), and an antibody to a part or the entirety of the constituents of the protein complex, in which both ends of the non-peptide polymer are respectively linked to the immunoglobulin Fc region and the physiologically active polypeptide, but is not limited thereto.

Still another aspect of the present invention provides a pharmaceutical composition with improved *in vivo* durability and stability of a physiologically active polypeptide, which comprises the above-described protein complex or a protein complex prepared by the preparation method as an active ingredient.

The pharmaceutical composition may further comprise a pharmaceutically acceptable excipient.

The pharmaceutical composition of the present invention may be administered via various routes including oral, transdermal, subcutaneous, intravenous, and intramuscular routes, and preferably, in the form of an injectable formulation. Additionally, the pharmaceutical composition of the present invention may be formulated by a method known in the art in order to provide rapid, long-acting, or delayed release of the active ingredient after its administration to a mammal. The formulation may be prepared in the form of a tablet, a pill, a powder, a sachet, an elixir, a suspension, an emulsion, a solution, a syrup, an aerosol, a soft or hard gelatin capsule, a sterile injectable solution, a sterile powder, *etc.* Examples of carriers, excipients, and diluents suitable for formulation may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, *etc.* The pharmaceutical composition may further comprise a filler, an anticoagulant, a lubricant, a humectant, a flavoring agent, an emulsifying agent, a preservative, *etc.*

The actual dose of the protein complex of the present invention may be determined by the types of the physiologically active polypeptide as an active component along with several related factors (*e.g*., disease to be treated, administration routes, the patient's age, sex, body weight, and severity of the disease, *etc*.). Since the protein complex of the present invention has excellent *in vivo* activity and durability in the blood, it can significantly reduce the administration dose, number of administration, and administration frequency of a peptide drug comprising the protein complex of the present invention.

Hereinafter, the present invention will be described in more detail with reference to the following Examples. However, the following Examples are only for illustrating the present invention, but the scope of the present invention is not limited thereto.

### Example 1: Preparation of complex in which acylated native insulin and immunoglobulin Fc are linked by non-peptide polymer

A complex in which acylated native insulin is linked to an immunoglobulin Fc fragment by a non-peptide polymer was prepared.

Specifically, acylated insulin in which native insulin is linked to 4-carboxy-3-fluorophenylboronic acid (PBA-F) through 12-dodecanoic acid (hereinafter, abbreviated as "Ins-PBA-F") was synthesized using the method disclosed in the cited reference and it was used for the preparation of the complex (Chou et al., PNAS Vol. 112, No. 8, p. 2401 to 2406).

Specifically, starting the synthesis from 12-dodecanoic acid, the ε-amino group of a lysine residue (*i.e.,* the 29^{th} amino acid of the insulin B-chain) was linked to the carboxy group of the 12-dodecanoic acid by an amide bond using a dicyclohexylcarbodiimide/N-hydroxysuccinimide (DCC/NHS) coupling reaction at normal conditions.

The preparation method of 12-(4-borono-2-fluorobenzamido)dodecanoic acid used for acylation of insulin is as follows.

(3-Fluoro-4-((12-methoxy-12-oxododecyl)carbamoyl)phenyl)boronic acid (5.15 g, 13.04 mmol) and lithium hydroxide (1.56 g, 65.20 mmol) were dissolved in a MeOH/water (3:1) solution (80 mL). The reaction was performed overnight while stirring in the presence of nitrogen and the solvent was removed under vacuum. Then, the reactants were dissolved in water, acidified to pH 1 with 1 N HCl, and the precipitate was obtained by filtration. A white product was obtained by column chromatography (0 to 20% MeOH in DCM) (3.23 g, 8.48 mmol, 65%). ¹H NMR (CD₃OD) δ7.73 (m, 3H), δ3.35 (t, 2H), δ2.26 (t, 2H), δ1.60 (m, 4H), δ1.31 (m, 14H) ¹³C NMR (CD₃OD) δ177.0, 129.7, 127.7, 121.1, 121.0, 40.3, 34.2, 29.9, 29.7, 29.5, 27.3, 25.4 HRMS: calculated 381.2123; found: 381.2124

Acylation of insulin was performed by the following method.

The 12-(4-borono-2-fluorobenzamido)dodecanoic acid, or myristic acid (20.67 µmol), N, N'-dicyclohexylcarbodiimide (4.26 mg, 20.67 µmol), and N-hydroxysuccinimide (2.38 mg, 20.67 µmol) were dissolved in tetrahydrofuran (1 mL) and mixed at room temperature for 4 hours.

Native insulin (100 mg, 17.23 µmol) was dissolved in 0.1 M Na₂CO3 (1 mL), and then added to the 12-(4-borono-2-fluorobenzamido)dodecanoic acid reactant, which was under reaction while stirring. After an hour, .2 M CH₃NH₂ (1 mL) and 6 N HCl (26 µL) were added to the reactant. The resulting reaction solution and precipitate were centrifuged and the pellet was resuspended in DMSO (2 mL). The product was purified by reverse phase HPLC (Atlantis C18 column (250 mm × 10 mm, 5 µm; Waters)). Fractions were collected and lyophilized under reduced pressure to give a white product. The product was confirmed by deconvolution ESI using QSTAR hybrid Q-TOF (Koch Institute Swanson Biotechnology Center Biopolymers and Proteomics core).

The amount obtained is as follows: Ins-PBA-F, predicted amount: 6,153, actual amount 6,115.

In this acylated insulin, p-carboxyphenylboronic acid was linked to the amino group of 12-dodecanoic acid and the ε-amino group of a lysine residue (*i.e.,* the 29^{th} amino acid of the insulin B-chain) was linked to the carboxy group of the 12-dodecanoic acid by an amide bond. The schematic diagram thereof is shown in FIG. 4. The term "acylated native insulin" is used interchangeably with "(PBA-F)-fatty acid-native insulin".

Then, to link 3.4 K PropionALD(2) PEG (a polyethylene glycol with a molecular weight of 3.4 kDa modified to have a propionaldehyde group at each end: NOF, Japan) to the N-terminus of Ins-PBA-F, Ins-PBA-F and 3.4K PropionALD(2) PEG were mixed at a molar ratio of 1:4 and the concentration of Ins-PBA-F was set at 5 mg/mL, and reacted at a temperature of 4□ to 8□ for about two hours. In particular, the reaction was performed in an environment in which a reducing agent, sodium cyanoborohydride (NaCNBH₃), was added to a mixed solvent of 50 mM sodium citrate buffer (pH 5.0) and isopropanol. Upon completion of the reaction, the reactants were applied to SP Sepharose High Performance (GE, USA) using a concentration gradient of a buffer containing 20 mM sodium citrate (pH 3.0) and ethanol, and 0.25 M KCl to purify the monoPegylated (PBA-F)-fatty acid-native insulin.

Then, to link the purified monoPegylated Ins-PBA-F to the N-terminus of an immunoglobulin Fc, the reaction was performed at a molar ratio of 1:1.2 between the reactants with the concentration of the total protein set at about 20 mg/mL, at a temperature of about 20□ to 25□ for about 16 hours. The reaction was performed in an environment in which sodium cyanoborohydride (*i.e.,* a reducing agent) was added to 100 mM HEPES buffer (pH8.2) and sodium chloride. Upon completion of the reaction, the reactants were applied to Source15Q (GE, USA) using a concentration gradient of 20 mM Tris (pH 7.5) and 0.15 M sodium chloride, and applied to SourceISO (GE, USA) using a concentration gradient of ammonium sulfate and 20 mM Tris (pH 7.5), and thereby the complex in which Ins-PBA-F was linked to the immunoglobulin Fc through PEG by a covalent bond was purified.

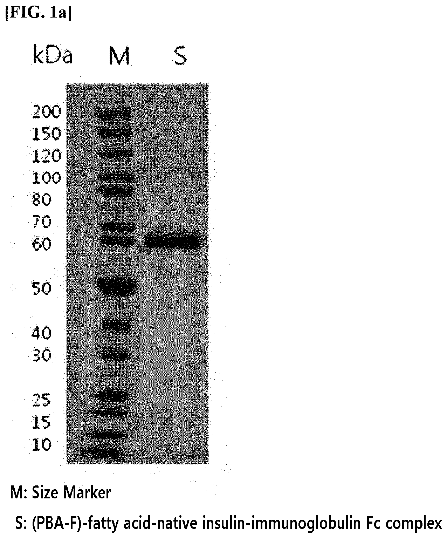

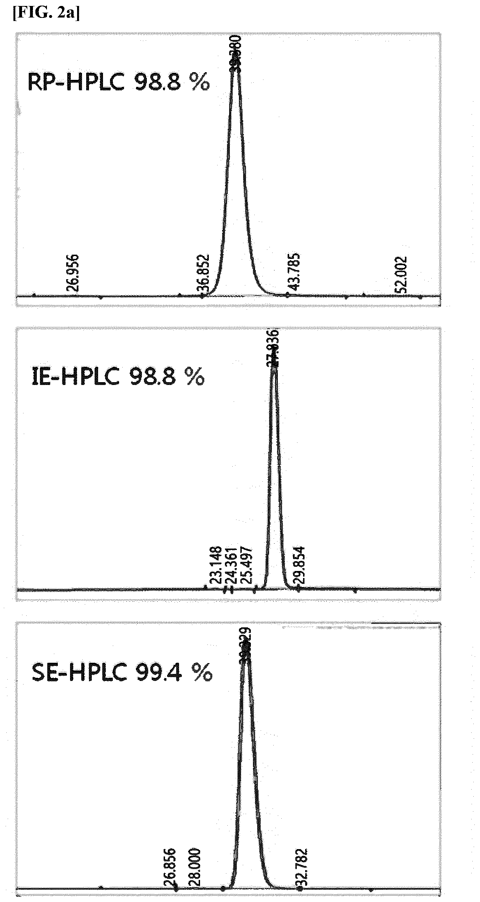

The purified complex was analyzed for purity using IE-HPLC, SE-HPLC, RP-HPLC, and SDS-PAGE and was confirmed to have a purity of at least 95% (FIGS. 1a and 2a).

### Example 2. Preparation of complex in which acylated insulin analog and immunoglobulin Fc are linked by non-peptide polymer

A complex in which an insulin analog is linked to an immunoglobulin Fc fragment by a non-peptide polymer was prepared. As the insulin analog, the insulin analogs, in which amino acids in the A-chain or B-chain of insulin are modified described in Examples of International Patent Publication No. WO 2014/133324, were used.

Table 1 shows the change in sequences of amino acids of the A- or B-chain of each of the insulin analogs and the analog names. That is, Analog 1 shows a case where the 1^{st} amino acid, glycine, of the A-chain is substituted with alanine, and Analog 4 shows a case where the 8^{th} amino acid, glycine, of the B-chain is substituted with alanine.

**[Table 1]**

| Analogs | Change of Sequence |
|---|---|
| Analog 1 | A¹G -> A |
| Analog 2 | A²I -> A |
| Analog 3 | A¹⁹Y -> A |
| Analog 4 | B⁸G -> A |
| Analog 5 | B²³G -> A |
| Analog 6 | B²⁴F -> A |
| Analog 7 | B²⁵F -> A |
| Analog 8 | A¹⁴Y -> E |
| Analog 9 | A¹⁴Y -> N |

Table 2 below shows the DNA sequences and protein sequences of specific insulin Analogs 1 to 9.

**[Table 2]**

| Analog | Sequence | | SEQ ID NO |
|---|---|---|---|
| Analog 1 | DNA | TTC GTT AAC CAA CAC TTG TGT GGC TCA CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GCG ATT GTG GAA CAA TGC TGT ACC AGC ATC TGC TCC CTC TAC CAG CTG GAG AAC TAC TGC AAC | 1 |
| | Protein | Phe Val Asn Gln His Leu Cys Gly Ser His Leu Val Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Ala Ile Val Glu Gln Cys Cys Thr Ser Ile Cys Ser Leu Tyr Gln Leu Glu Asn Tyr Cys Asn | 2 |
| Analog 2 | DNA | TTC GTT AAC CAA CAC TTG TGT GGC TCA CAC CTG GTG GAA GCT CTC TAC CTA GTG | 3 |
| | | TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC GCG GTG GAA CAA TGC TGT ACC AGC ATC TGC TCC CTC TAC CAG CTG GAG AAC TAC TGC AAC | |
| | Protein | Phe Val Asn Gln His Leu Cys Gly Ser His Leu Val Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ala Val Glu Gln Cys Cys Thr Ser Ile Cys Ser Leu Tyr Gln Leu Glu Asn Tyr Cys Asn | 4 |
| Analog 3 | DNA | TTC GTT AAC CAA CAC TTG TGT GGC TCA CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC GCG TGC AAC | 5 |
| | Protein | Phe Val Asn Gln His Leu Cys Gly Ser His Leu Val Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Ala Cys Asn | 6 |
| Analog 4 | DNA | TTC GTT AAC CAA CAC TTG TGT GCG TCA CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC TAC TGC AAC | 7 |
| | Protein | Phe Val Asn Gln His Leu Cys Ala Ser His Leu Val Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | 8 |
| Analog 5 | DNA | TTC GTT AAC CAA CAC TTG TGT GGC TCA CAC CTG GTG GAA GCT CTC TAC CTA GTG TGC GGG GAA CGA GCG TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC TAC TGC AAC | 9 |
| | Protein | Phe Val Asn Gln His Leu Cys Gly Ser His Leu Val Glu Ala Leu Tyr Leu Val Cys Gly Glu Arg Ala Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro | 10 |
| | | Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | |
| Analog 6 | DNA | TTC GTT AAC CAA CAC TTG TGT GGC TCA CAC CTG GTG GAA GCT CTC TAC CTA GTG TGC GGG GAA CGA GGC GCG TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC TAC TGC AAC | 11 |
| | Protein | Phe Val Asn Gln His Leu Cys Gly Ser His Leu Val Glu Ala Leu Tyr Leu Val Cys Gly Glu Arg Gly Ala Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | 12 |
| Analog 7 | DNA | TTC GTT AAC CAA CAC TTG TGT GGC TCA CAC CTG GTG GAA GCT CTC TAC CTA GTG TGC GGG GAA CGA GGC TTC GCG TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC TAC TGC AAC | 13 |
| | Protein | Phe Val Asn Gln His Leu Cys Gly Ser His Leu Val Glu Ala | 14 |
| | | Leu Tyr Leu Val Cys Gly Glu Arg Gly Phe Ala Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | |
| Analog 8 | DNA | TTC GTT AAC CAA CAC TTG TGT GGC TCA CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC GAA CAG CTG GAG AAC TAC TGC AAC TGA | 15 |
| | Protein | Phe Val Asn Gln His Leu Cys Gly Ser His Leu Val Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Glu Gln Leu Glu Asn Tyr Cys Asn | 16 |
| Analog 9 | DNA | TTC GTT AAC CAA CAC TTG TGT GGC TCA CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT | 17 |
| | | ACC AGC ATC TGC TCC CTC AAC CAG CTG GAG AAC TAC TGC AAC TGA | |
| | Protein | Phe Val Asn Gln His Leu Cys Gly Ser His Leu Val Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Asn Gln Leu Glu Asn Tyr Cys Asn | 18 |

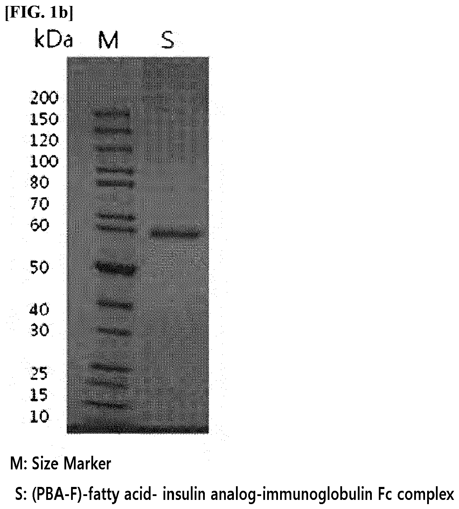

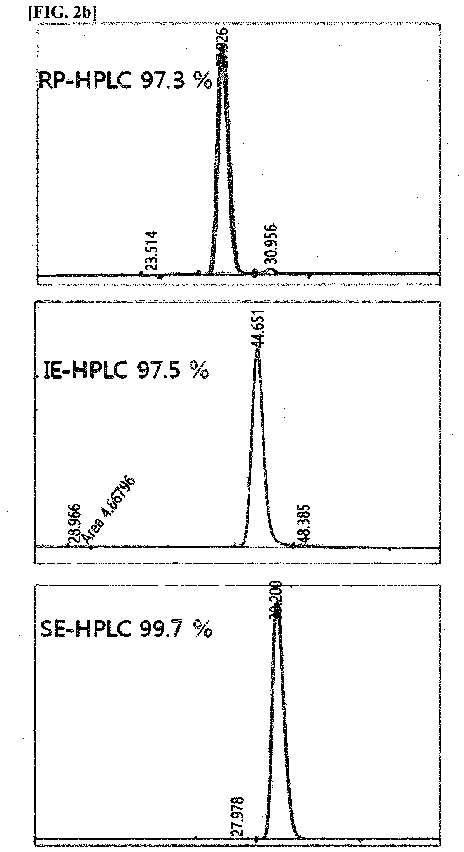

The experimental procedure described in Example 1 was followed, except that insulin analogs were used instead of native insulin, and the analog complexes showed a purity of 95% or higher (FIGS. 1b and 2b). The term acylated insulin analog can be used interchangeably with (PBA-F)-fatty acid-insulin analog.

### Example 3. Comparison of in vitro activity between acylated native insulin complex and acylated insulin analog complex

The protein complex of the present invention, in which a physiologically active polypeptide and an immunoglobulin Fc region are linked to a non-peptide polymer linker, was compared with the existing protein complexes with regard to their activities, to confirm whether the activity of the protein complex of the present invention is excellent because the relative activity is reduced.

Specifically, to confirm the *in vitro* activity of the long-acting native insulin complex and the long-acting insulin analog protein complex, in which the (PBA-F)-fatty acid is linked prepared in Examples 1 and 2, a method of measuring the phosphorylation of insulin receptors using a Chinese hamster ovary (CHO) cell line, which has overexpressed human insulin receptors, was employed.

The cell line was subcultured two or three times a week, and the CHO cells in which the human insulin receptors were expressed were seeded into each well of a 96-well plate at a density of 5 × 10⁴ cells/well and cultured for 24 hours, respectively.

For confirmation and comparison of the activity of the long-acting insulin analogs, human insulin was subjected to a 3-fold serial dilution from 344.4 nM to 0.2 nM, and the long-acting native insulin and the long-acting insulin analog were subjected to a 3-fold serial dilution from 20,037.1 nM to 9.2 nM. The cultured CHO cells in which the human insulin receptors were expressed were washed with KRB buffer, and each of the materials which underwent serial dilutions was added in an amount of 100 µL to the CHO cells and incubated in a CO₂ incubator at 37 □ for 10 minutes. Then, 25 µL of cell lysis buffer was added to each well to lyse the cells. The cell lysates from each well were subjected to the Phospho-Insulin Receptor β Sandwich ELISA kit (Cell signaling, USA) to measure the phosphorylated insulin receptors, and EC₅₀ values were calculated therefrom and *in vitro* activitiy was compared. The EC₅₀ values and the activity relative to that of human insulin for each material by two repeated experiments are shown in Table 3 below. The activity of the complexes of Examples 1 and 2 was respectively compared with that of a native insulin-immunoglobulin Fc complex (having the same structure as that of Example 1) and that of an insulin analog-immunoglobulin Fc complex (having the same structure as that of Example 2) except that unacylated native insulin and unacylated insulin analog were used. The complexes for comparison purposes except Examples 1 and 2 were prepared as described in International Patent Publication No. WO2014133324. Based on the human native insulin, the *in vitro* activity of the native insulin-immunoglobulin Fc complex, insulin analog-immunoglobulin Fc complex, acylated native insulin- immunoglobulin Fc complex of Example 1, and acylated insulin analog-immunoglobulin Fc complex of Example 2 was 5.4%, 2.1%, 2.8%, and 2.2%, respectively.

**[Table 3]**

| Test Material | ECso (nM) | % Activity vs. Human Native Insulin |
|---|---|---|
| Native Insulin | 14.2 ± 0.8 | 100 |
| Native Insulin-Immunoglobulin Fc Complex | 264.8 ± 31.8 | 5.4 ± 0.3 |
| Insulin Analog-Immunoglobulin Fc Complex | 675.2 ± 90.2 | 2.1 ± 0.1 |
| Acylated Native Insulin Complex of Example 1 | 520.1 ± 68.8 | 2.8 ± 0.2 |
| Acylated Insulin Analog Complex of Example 2 | 661.4 ± 7.0 | 2.2 ± 0.1 |

The above results suggest that the long-acting protein complex modified with a non-peptide polymer newly developed in the present invention shows an *in vitro* activity comparable to those of existing protein complexes, and thus, the modification by a non-peptide polymer does not cause any problem with regard to its *in vitro* activity.

In this regard, the present inventors performed the following *in vivo* efficacy experiment to confirm whether the long-acting protein complex comprising a physiologically active polypeptide modified with a non-peptide polymer can actually exhibit improved *in vivo* activity.

### Example 4. Comparison of in vivo activity between acylated native insulin complex and acylated insulin analog complex

The protein complex of the present invention, in which a physiologically active polypeptide and an immunoglobulin Fc region are linked to a non-peptide polymer (*i.e.,* a linker), was compared with the existing protein complexes with regard to their activities, to confirm whether the protein complex of the present invention exhibits an improved *in vivo* durability compared to the existing protein complexes.

Streptozotocin (STZ)-induced diabetic mice, which are widely used as a diabetic animal model, were used for this study. To induce diabetic mice, 7-week-old C57BL/6 mice were fasted for 4 hours, and administered with 50 mg/kg of STZ (10 mM citrate buffer, pH 4.5) once. After 4-7 days of STZ administration, the induction of diabetes was confirmed using a blood glucose meter (OneTouch® Ultra®, LifeScan, Inc., USA), and only the diabetic mice with blood glucose levels of about 400 mg/mL were used for the experiment.

The diabetic mice were divided into following groups and administered with the test material: Group 1: Vehicle (single injection), Group 2: an insulin analog-immunoglobulin Fc complex 100 nmol/kg (single injection), Group 3: (PBA-F)-fatty acid-insulin analog-immunoglobulin Fc complex 100 nmol/kg (single injection), Group 4: (PBA-F)-fatty acid-native insulin-immunoglobulin Fc complex 100 nmol/kg (single injection). During the study, the blood glucose levels of the mice were measured at 0, 2, 4, and 8 hours, and 1, 1.25, 2, 2.25, 3, 4, 5, 6, 7, 8, 9, and 10 days after the administration of the test material. The AUC was calculated using the changes in blood glucose levels over the 10 days of measurement. For statistical treatment, the excipient group and the test group were compared using one-way ANOVA.

The changes in blood glucose levels and calculated AUC values are shown in FIG. 3. Compared to the group administered with a vehicle, the insulin analog-immunoglobulin Fc complex, the (PBA-F)-fatty acid-insulin analog-immunoglobulin Fc complex, and the (PBA-F)-fatty acid-native insulin-immunoglobulin Fc complex showed a hypoglycemic effect of -11%, - 27%, and -8%, respectively.

The above results suggest that the long-acting protein complex modified with a non-peptide polymer newly developed in the present invention shows an *in vitro* activity comparable to those of existing protein complexes, and thus, the modification by a non-peptide polymer does not cause any problem with regard to its *in vivo* activity.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A protein complex, wherein a physiologically active polypeptide modified with a fatty acid molecule and an immunoglobulin Fc region are linked through a non-peptide polymer linker.

2. The protein complex of claim 1, wherein the physiologically active polypeptide modified with a fatty acid molecule is one in which at least one fatty acid molecule is linked to a physiologically active polypeptide, wherein the fatty acid molecule used in modification is not linked to the immunoglobulin Fc region

3. The protein complex of claim 1 or claim 2, wherein the fatty acid molecule which has modified the physiologically active polypeptide is a fatty acid or fatty acid derivative.

4. The protein complex of any one of claims 1 to 3, wherein the non-peptide polymer linker, through reactive groups at both ends thereof, is linked by a covalent bond to the physiologically active polypeptide modified with a fatty acid molecule and the immunoglobulin Fc region, respectively.

5. The protein complex of any one of claims 1 to 4, wherein the immunoglobulin Fc region is aglycosylated.

6. The protein complex of any one of claims 1 to 5, wherein the immunoglobulin Fc region consists of one to four domains selected from the group consisting of CH1, CH2, CH3, and CH4 domains.

7. The protein complex of any one of claims 1 to 6, wherein the immunoglobulin Fc region further comprises a hinge region.

8. The protein complex of any one of claims 1 to 7, wherein the immunoglobulin Fc region is an immunoglobulin Fc fragment selected from the group consisting of the Fc regions of IgG, IgA, IgD, IgE, IgM, a combination thereof, and a hybrid thereof.

9. The protein complex of any one of claims 1 to 8, wherein the immunoglobulin Fc region is selected from the group consisting of the Fc regions of IgG1, IgG2, IgG3, IgG4, a combination thereof, and a hybrid thereof.

10. The protein complex of any one of claims 1 to 9, wherein the immunoglobulin Fc fragment is a dimer or multimer consisting of single chain immunoglobulins consisting of domains having the same origin.

11. The protein complex of any one of claims 1 to 10, wherein the immunoglobulin Fc fragment is an IgG4 Fc fragment.

12. The protein complex of any one of claims 1 to 11, wherein the immunoglobulin Fc fragment is a human aglycosylated IgG4 Fc fragment.

13. The protein complex of any one of claims 1 to 12, wherein the non-peptide polymer linker is selected from the group consisting of polyethylene glycol, polypropylene glycol, an ethylene glycol-propylene glycol copolymer, polyoxyethylated polyol, polyvinyl alcohol, polysaccharide, dextran, polyvinyl ethyl ether, a biodegradable polymer, a lipid polymer, a fatty acid, a fatty acid derivative, chitin, hyaluronic acid, and a combination thereof.

14. The protein complex of any one of claims 1 to 13, wherein the non-peptide polymer linker is a fatty acid, a fatty acid derivative, or polyethylene glycol.

15. The protein complex of any one of claims 1 to 14, wherein both ends of the non-peptide polymer linker are respectively linked to the N-terminus, a lysine residue, or an arginine residue of the immunoglobulin Fc region and the N-terminus, the C-terminus, a lysine residue, a histidine residue, or a cysteine residue of the physiologically active polypeptide; or a free reactive group of each terminus or residue thereof.

16. The protein complex of any one of claims 1 to 15, wherein the physiologically active polypeptide is selected from the group consisting of hormone, cytokine, enzyme, antibody, growth factor, transcription factor, blood coagulation factor, vaccine, structural protein, ligand protein, and receptor.

17. The protein complex of any one of claims 1 to 16, wherein the physiologically active polypeptide is selected from the group consisting of human growth hormone, growth hormone-releasing hormone, growth hormone-releasing peptide, interferons, interferon receptors, colony-stimulating factors, glucagon-like peptides (GLP-1, *etc*.), exendins (Exendin-4, *etc*.), oxyntomodulin, G-protein-coupled receptor, interleukins, interleukin receptors, enzymes, interleukin-coupled proteins, cytokine-coupled proteins, macrophage activating factor, macrophage peptide, B cell factor, T cell factor, protein A, allergy-inhibiting factor, cell necrosis glycoprotein, immunotoxin, lymphotoxin, tumor necrosis factor, tumor-inhibiting factor, transforming growth factor, α-1 antitrypsin, albumin, α-lactalbumin, apolipoprotein-E, erythropoietin, high-glycosylated erythropoietin, angiopoeitin, hemoglobin, thrombin, thrombin receptor-activating peptides, thrombomodulin, blood coagulation factors VII, VIIa, VIII, IX, and XIII, plasminogen activator, fibrin-binding peptides, urokinase, streptokinase, hirudin, protein C, C-reactive protein, renin inhibitor, collagenase inhibitor, superoxide dismutase, leptin, platelet-derived growth factor, epithelial growth factor, epidermal growth factor, angiostatin, angiotensin, bone morphogenetic growth factor, bone morphogenetic-stimulating protein, calcitonin, insulin, atriopeptin, cartilage-inducing factor, elcatonin, connective tissue-activating factor, tissue factor pathway inhibitor, follicle-stimulating hormone, luteinizing hormone, luteinizing hormone-releasing hormone, nerve growth factors, parathyroid hormone, relaxin, secretin, somatomedin, insulin-like growth factor, adrenocortical hormone, glucagon, cholecystokinin, pancreatic polypeptide, gastrin-releasing peptide, corticotropin-releasing factor, thyroid-stimulating hormone, autotaxin, lactoferrin, myostatin, receptors, receptor antagonist, cell surface antigen, virus-derived vaccine antigen, monoclonal antibody, polyclonal antibody, antibody fragments, and a derivative thereof.

18. The protein complex of any one of claims 1 to 17, wherein the physiologically active polypeptide is selected from the group consisting of human growth hormone, interferon-alpha, granulocyte colony-stimulating factor, erythropoietin, blood coagulation factor, insulin, oxyntomodulin, glucagon-like peptides, exendins, and a derivative or analog thereof.

19. The protein complex of any one of claims 1 to 18, wherein the physiologically active polypeptide is insulin or an analog thereof, in which the fatty acid molecule which has modified the physiologically active polypeptide is a fatty acid or fatty acid derivative, and the non-peptide polymer linker is polyethylene glycol.

20. The protein complex of claim 18 or claim 19, wherein the insulin analog is one in which any one, which is selected from the group consisting of the 8^{th} amino acid of insulin B-chain, the 23^{rd} amino acid of insulin B-chain, the 24^{th} amino acid of insulin B-chain, the 25^{th} amino acid of insulin B-chain, the 1^{st} amino acid of insulin A-chain, the 2^{nd} amino acid of insulin A-chain, and the 19^{th} amino acid of insulin A-chain, is substituted with alanine; or the 14^{th} amino acid of insulin A-chain is substituted with glutamic acid or asparagine.

21. A method of preparing the protein complex of any one of claims 1 to 20, comprising:
(a) linking a physiologically active polypeptide, which is in a form where at least one fatty acid molecule is linked thereto, and at least one immunoglobulin Fc region by a covalent bond through a non-peptide polymer linker having a reactive group at both ends to prepare a protein complex; and
(b) separating the protein complex prepared in Step (a), which essentially comprises a physiologically active polypeptide, which is in a form where a fatty acid molecule is linked thereto, and an immunoglobulin Fc region that are linked by a covalent bond through the non-peptide polymer linker, wherein the non-peptide polymer linker is linked to the immunoglobulin Fc fragment.

22. The method of claim 21, wherein the reactive group of the non-peptide polymer or fatty acid molecule is selected from the group consisting of an aldehyde group, a maleimide group, and a succinimide derivative.

23. The method of claim 22, wherein the aldehyde group is a propionaldehyde group or butyraldehyde group.

24. The method of claim 22, wherein the succinimide derivative is succinimidyl carboxymethyl, succinimidyl valerate, succinimidyl methylbutanoate, succinimidyl methylpropionate, succinimidyl butanoate, succinimidyl propionate, N-hydroxysuccinimide, or succinimidyl carbonate.

25. The method of any one of claims 21 to 24, wherein the non-peptide polymer linker has an aldehyde group, a maleimide reactive group, or a succinimide derivative as a reactive group at each end, respectively.

26. The method of any one of claims 21 to 25, wherein the fatty acid molecule has an aldehyde group, a maleimide reactive group, or a succinimide derivative as a reactive group at each end, respectively.

27. The method of any one of claims 21 to 26, wherein Step (a) comprises:
(a1) linking one end of the non-peptide polymer linker to any one of the immunoglobulin Fc region or the physiologically active polypeptide, which is in a form where a fatty acid molecule is linked thereto, by a covalent bond to prepare a protein conjugate; and
(a2) separating the conjugate prepared in Step (a1) and linking the other end of the physiologically active polypeptide, which is in a form where a fatty acid molecule is linked thereto, of the separated conjugate to the other of the immunoglobulin Fc region or the physiologically active polypeptide, by a covalent bond.

28. The method of claim 27, wherein, in Step (a1), the reaction molar ratio between the physiologically active polypeptide, which is in a form where a fatty acid molecule is linked thereto, and the non-peptide polymer linker is in a range of 1:1 to 1:30, and the reaction molar ratio between the immunoglobulin Fc fragment and the non-peptide polymer is in a range of 1:1 to 1:20.

29. The method of claim 28, wherein Step (a1) is performed at a pH between 4.0 and 9.0.

30. The method of claim 27, wherein Step (a1) is performed at a temperature between 4.0°C and 25°C.

31. The method of any one of claims 27 to 30, wherein, in Step (a1), the reaction concentration of the immunoglobulin Fc region or the physiologically active polypeptide, which is in a form where a fatty acid molecule is linked thereto, is in a range of 0.1 mg/mL to 100 mg/mL.

32. The method of claim 27, wherein, in Step (a2), the reaction molar ratio between the conjugate and the immunoglobulin Fc region or the physiologically active polypeptide is in a range of 1:0.1 to 1:20.

33. The method of claim 27 or claim 32, wherein Step (a2) is performed at a pH between 4.0 and 9.0.

34. The method of claim 27 or claim 32, wherein Step (a2) is performed at a temperature between 4.0°C and 25°C.

35. The method of any one of claims 27 to 34, wherein, in Step (a2), the reaction concentration of the immunoglobulin Fc region or the physiologically active polypeptide, which is in a form where a fatty acid molecule is linked thereto, is in a range of 0.1 mg/mL to 100 mg/mL.

36. The method of any one of claims 27 to 35, wherein Step (a1) and Step (a2) are performed in the presence of a reducing agent.

37. The method of claim 36, wherein the reducing agent is selected from the group consisting of sodium cyanoborohydride (NaCNBH₃), sodium borohydride, dimethylamine borate, and pyridine borate.

38. The method of claim 27, wherein, in Step (a2), the conjugate is separated by performing a single purification method or a combination of multiple purification methods selected from the group consisting of anion exchange chromatography, cation exchange chromatography, hydrophobic chromatography, affinity chromatography, and size exclusion chromatography.

39. The method of claim 38, wherein the functional group of the anion exchange chromatography resin is any one selected from the group consisting of quaternary ammonium (Q), quaternary aminoethyl (QAE), diethylaminoethyl (DEAE), polyethylene imine (PEI), dimethylaminomethyl (DMAE), and trimethylaminoethyl (TMAE).

40. The method of claim 37, wherein the functional group of the cation exchange chromatography resin is any one selected from the group consisting of methylsulfonate (S), sulfopropyl (SP), carboxymethyl (CM), polyaspartic acid, sulfoethyl (SE), sulfopropyl (SP), phosphate (P), and sulfonate (S).

41. The method of claim 38, wherein the functional group of the hydrophobic chromatography resin is any one selected from the group consisting of phenyl, octyl, (iso)propyl, butyl, and ethyl.

42. The method of claim 38, wherein the functional group of the affinity chromatography resin is any one selected from the group consisting of protein A, heparin, blue, benzamidine, metal ions (cobalt, nickel, and copper), and an antibody to a part or the entirety of constituents of the protein complex, in which both ends of the physiologically active polypeptide, which is in a form where a fatty acid molecule is linked thereto, are respectively linked to the immunoglobulin Fc region and the physiologically active polypeptide.

43. The method of claim 38, wherein the resin of the size exclusion chromatography is any one selected from the group consisting of Superdex, Sephacryl, Superpose, and Sephadex.

44. The method of claim 21, wherein, in Step (b), the protein complex is separated by performing a single purification method or a combination of multiple purification methods selected from the group consisting of anion exchange chromatography, cation exchange chromatography, hydrophobic chromatography, affinity chromatography, and size exclusion chromatography.

45. The method of claim 44, wherein the functional group of the anion exchange chromatography resin is any one selected from the group consisting of quaternary ammonium (Q), quaternary aminoethyl (QAE), diethylaminoethyl (DEAE), polyethylene imine (PEI), dimethylaminomethyl (DMAE), and trimethylaminoethyl (TMAE).

46. The method of claim 44, wherein the functional group of the cation exchange chromatography resin is any one selected from the group consisting of methylsulfonate (S), sulfopropyl (SP), carboxymethyl (CM), polyaspartic acid, sulfoethyl (SE), sulfopropyl (SP), phosphate (P), and sulfonate (S).

47. The method of claim 44, wherein the functional group of the hydrophobic chromatography resin is any one selected from the group consisting of phenyl, octyl, (iso)propyl, butyl, and ethyl.

48. The method of claim 44, wherein the functional group of the affinity chromatography resin is any one selected from the group consisting of protein A, heparin, blue, benzamidine, metal ions (cobalt, nickel, and copper), and an antibody to a part or the entirety of constituents of the protein complex, in which both ends of the non-peptide polymer are respectively linked to the immunoglobulin Fc region and the physiologically active polypeptide.

49. The method of claim 44, wherein the resin of the size exclusion chromatography is any one selected from the group consisting of Superdex, Sephacryl, Superpose, and Sephadex.

50. The method of claim 21, wherein, in Step (b), the protein complex, in which a physiologically active polypeptide, which is in a form where a fatty acid molecule is linked thereto, is linked to an immunoglobulin Fc region through the N-terminus of the immunoglobulin Fc region, is separated.

51. A method of preparing the protein complex of any one of claims 1 to 20, comprising:
(a') linking one end of the non-peptide polymer to any one of the immunoglobulin Fc region or the physiologically active polypeptide, which is in a form where a fatty acid molecule is linked thereto, by a covalent bond to prepare a conjugate,
wherein the reaction molar ratio between the physiologically active polypeptide, which is in a form where a fatty acid molecule is linked thereto, and the non-peptide polymer is in a range of 1:1 to 1:30; the reaction molar ratio between the immunoglobulin Fc fragment and the non-peptide polymer linker is in a range of 1:1 to 1:20; a reducing agent is contained at a concentration of 1 mM to 100 mM; the reaction is performed at a pH between 4.0 and 9.0 and at a temperature between 4.0°C and 25°C; and the reaction concentration of the immunoglobulin Fc region or the physiologically active polypeptide is in a range of 0.1 mg/mL to 100 mg/mL;
(b') separating the conjugate prepared in Step (a') and linking the other end of the non-peptide polymer linker of the separated conjugate to the other of the immunoglobulin Fc region and the physiologically active polypeptide, by a covalent bond,
wherein the reaction molar ratio between the conjugate and the immunoglobulin Fc region or the physiologically active polypeptide is in a range of 1:0.1 to 1:20; a reducing agent is contained at a concentration of 1 mM to 100 mM; the reaction is performed at a pH between 4.0 and 9.0 and at a temperature between 4.0°C and 25°C; and the reaction concentration of the immunoglobulin Fc region or the physiologically active polypeptide, which is in a form where a fatty acid molecule is linked thereto, is in a range of 0.1 mg/mL to 100 mg/mL; and
(c') separating the protein complex prepared in Step (b'), which essentially comprises a physiologically active polypeptide, which is in a form where a fatty acid molecule is linked thereto, a non-peptide polymer linker, and an immunoglobulin Fc region that are linked by a covalent bond, in which the non-peptide polymer linker is linked to the immunoglobulin Fc.

52. A pharmaceutical composition for improving *in vivo* durability and stability of a physiologically active polypeptide comprising the protein complex according to any one of claims 1 to 20 as an active ingredient.
